# EUROPEAN PATENT SPECIFICATION

(11) **EP 2 142 934 B1**
(45) Date of publication and mention of the grant of the patent: **26.10.2011**
(21) Application number: 08754072.0
(22) Date of filing: 03.04.2008
(51) Int. Cl.: G01N 33/68

(54) **PRION ASSAY**
PRIONENTEST
DOSAGE BIOLOGIQUE DU PRION

(30) Priority: 04.04.2007 US 921951 P; 21.02.2008 US 66704
(43) Date of publication of application: 13.01.2010
(73) Proprietor: Novartis AG, 4056 Basel (CH)
(72) Inventor: PERETZ, David, Emeryville, CA 94608 (US); YAM, Alice, Emeryville, CA 94608 (US)
(74) Representative: Marshall, Cameron John
(86) International application number: PCT/US2008/004454
(87) International publication number: WO 2008/124098

(56) References cited:
- WO-A-2006/076687
- WO-A-2006/088281
- KUCZIUS ET AL: "Immunoreactivity enhancement with chelators for increasing the detection sensitivity of human PrP<Sc> by Western blotting" NEUROCHEMISTRY INTERNATIONAL, PERGAMON PRESS, OXFORD, GB, vol. 50, no. 1, 9 December 2006 (2006-12-09), pages 102-108, XP005799261 ISSN: 0197-0186
- POLYMENIDOU ET AL: "Coexistence of multiple PrP<Sc> types in individuals with Creutzfeldt-Jakob disease" LANCET NEUROLOGY, LANCET PUBLISHING GROUP, LONDON, GB, vol. 4, no. 12, 1 December 2005 (2005-12-01), pages 805-814, XP005170086 ISSN: 1474-4422

## Description

### FIELD OF THE INVENTION

The invention relates to an assay method of detecting the presence of pathogenic prions in a sample. The invention also relates to kits comprising reagents used in the assay method.

### BACKGROUND

Amyloid diseases are caused by the transition of soluble protein into an insoluble aggregated form. Evidence exists that this conversion is associated with a conformational change to the secondary and tertiary structure as well. There is increasing evidence that accumulation of protein deposits damage cells and tissues leading to diseases. Several diseases are associated with the deposition of specific proteins.

One group of conformational diseases is termed "prion diseases" or "transmissible spongiform encephalopathies (TSEs)." These diseases manifest in humans and animals. In humans, prion diseases include Creutzfeldt-Jakob disease (CJD), variant CJD (vCJD), Gerstmann-Straussler-Scheinker syndrome (GSS), Fatal Familial Insomnia, and Kuru (see, e.g., Isselbacher et al., eds. (1994). Harrison's Principles of Internal Medicine. New York: McGraw.Hill, Inc.; Medori et al. (1992) N. Engl. J. Med. 326: 444-9). In animals, prion diseases include sheep scrapie, bovine spongiform encephalopathy (BSE), transmissible mink encephalopathy, and chronic wasting disease of captive mule deer and elk (Gajdusek, (1990). Subacute Spongiform Encephalopathies: Transmissible Cerebral Amyloidoses Caused by Unconventional Viruses. In: Virology, Fields, ed., New York: Raven Press, Ltd. (pp. 2289-2324)).

Recently, the rapid spread of BSE and its correlation with elevated occurrence of TSEs in humans has led to increased interest in the detection ofTSEs in non-human mammals. The tragic consequences of accidental transmission of these diseases (see, e.g., Gajdusek, Infectious Amyloids, and Prusiner Prions In Fields Virology. Fields, et al., eds. Philadelphia: Lippincott-Ravin, Pub. (1996); Brown et al. Lancet, 340: 24-27 (1992)), decontamination difficulties (Asher et al. (1986) In: Laboratory Safety: Principles and Practices, Miller ed., (pp. 59-71) Am. Soc. Micro.), and concern about BSE (British Med. J. (1995) 311: 1415-1421) underlie the urgency of having both a diagnostic test that would identify humans and animals with TSEs and therapies for infected subjects.

Prions differ significantly from bacteria, viruses and viroids. The dominating hypothesis is that, unlike all other infectious pathogens, disease is caused by an abnormal conformation of the prion protein, which acts as a template and converts normal prion conformations into abnormal, aberrant conformations. The prion protein, or PrP, was first characterized in the early 1980s. (See, e.g., Bolton et al. (1982) Science 218: 1309-1311; Prusiner et al. (1982) Biochemistry 21: 6942-6950; McKinley et al. (1983) Cell 35: 57-62). Complete prion protein-encoding genes have since been cloned, sequenced and expressed in transgenic animals. (See, e.g., Basler et al. (1986) Cell 46: 417-428.) The normal, cellular form of the prion protein, which is also referred to as PrP^{C}, is a 33-35 kD protein of uncertain function and, in humans, is transcribed by a gene on the short arm of chromosome 20. The abnormally shaped prion protein is also referred to as a scrapie protein or a pathogenic prion protein (PrP^{Sc}). Deposition of PrP^{Sc} in the Central Nervous System (CNS) is associated with neuron degeneration and is always lethal. PrP^{Sc} is infectious and exposure to tissues containing infectivity could result in disease. Experimental inoculation of PrP^{Sc} into laboratory animals including primates, sheep, rodents, and transgenic mice resulted in transmission of prion disease. (See, e.g., Zhang et al. (1997) Biochem. 36(12): 3543-3553; Cohen & Prusiner (1998) Ann. Rev. Biochem. 67: 793-819; Pan et al. (1993) Proc. Natl. Acad. Sci. USA 90:10962-10966; Safar et al. (1993) J Biol. Chem. 268: 20276-20284.)

The substantially β-sheet structure of PrP^{Sc} as compared to the predominantly α-helical folded non-diseased forms of PrP^{C} has been revealed by optical spectroscopy and crystallography studies. (See, e.g., Wille et al. (2001) Proc. Nat'l Acad. Sci. USA 99: 3563-3568; Peretz et al. (1997) J. Mol. Biol. 273: 614-622; Cohen & Prusiner, (1999) 5: Structural Studies of Prion Proteins. In Prion Biology And Diseases, S. Prusiner, ed. Cold Spring Harbor, NY: Cold Spring Harbor Laboratory Press. (pp: 191-228.) The structural changes from PrP^{C} to PrP^{Sc} appear to be followed by alterations in biochemical properties: PrP^{C} is soluble in non-denaturing detergents, PrP^{Sc} is insoluble and forms oligomers that are composed of as many as 1000 molecules; PrP^{C} is readily digested by proteases, while PrP^{Sc} is partially resistant, resulting in the formation of an amino-terminally truncated fragment known as "PrP 27-30" (27-30 kDa), "PrPres"or "PK-resistant" (proteinase K-resistant) form or core, which corresponds to the PrP fragment of amino acid residues from about 90 to about 231, numbered according to the human or Syrian Hamster PrP full-length sequence including the signal peptide. (See, e.g., Baldwin et al. (1995) J. Biol. Chem. 270:19197-19200; Prusiner et al. (1982) Biochemistry 21:6942-6950; Prusiner et al. (1983) Cell 35:349-358; Collinge, J. and Palmer, M.S. (1997) Prion Diseases, Oxford University Press, New York.) Additionally, PrP^{Sc} can convert PrP^{C} to the pathogenic conformation. (See, e.g., Kaneko et al. (1995) Proc. Nat'l Acad. Sci. USA 92:11160-11164; Caughey (2003) Br Med Bull. 66: 109- 20; Telling et al. (1995) Cell 83:79-90; Kaneko et al. (1997) Proc. Natl. Acad. Sci. USA 94:10069-10074; DebBurman et al. (1997) Proc. Natl. Acad. Sci. USA 94: 13938-13943;.Horiuchi et al. (1999) EMBO J. 18:3193-3203; Horiuchi et al. (2000) Proc. Natl. Acad. Sci. USA 97: 5836-5841; Kocisko et al. (1994) Nature 370:471-474.)

Detection of the pathogenic isoforms of conformational disease proteins in living subjects, and samples obtained from living subjects, has proven difficult. Although detection of amyloids in general can be achieved with Congo red staining, this type of staining is inaccurate and not sensitive. Specific and high affinity detection of a given protein in the presence of other proteins like in cell, tissue or homogenate is usually done with antibodies that are specific to the targeted protein. Yet, proteins that share the same sequence but differ by conformation complicate discriminatory detection by antibodies. In fact, the majority of antibodies raised against PrP bind PrP^{C} or to both PrP^{C} and PrP^{Sc}. (See, e.g., Matsunaga et al. (2001) Proteins: Structure, Function and Genetics 44: 110-118). In addition, the aggregation of PrP^{Sc} reduces the effective epitope concentrations for an antibody; thereby hindering efficient binding of specific antibodies to PrP. Therefore, effective immunodetection of PrP^{Sc} necessitates dissociation and denaturation of PrP^{Sc} into PrP^{C}-like conformers while discriminating against the original PrP^{C} molecules.

Several published immunoassay methods were based on comparing the binding level of antibodies that only bind native PrP^{C} but not native PrP^{Sc} to the binding level of antibodies that bind both native PrP^{Sc} and PrP^{C} in order to determine the presence of PrP^{Sc}. (See, e.g., U.S. Patent Nos. 6,214,565 B1 and 6,406,864 B2.) Another immunoassay method was also based on comparing the antibody binding levels, but it involved a denaturing step to denature PrP^{Sc} and PrP^{C} before binding them to the same antibody as the one used to bind to native PrP^{C}. (See U.S. Patent No. 5,891,641.) However, there are limitations in these assay methods because, using these methods, effective antibody detection of PrP^{Sc} can only be obtained when the amount of PrP^{C} is very low. In an excess of PrP^{C}, e.g., when samples are taken from subjects in the early stages of prion disease, it is difficult or even impossible to observe an increase in immunodetection due to the presence of PrP^{Sc}. For example, detection of denatured PrP^{Sc} in plasma samples with 1000 times more PrP^{C} will not be possible because of the overwhelming signal from the detection of PrP^{C}. Thus, simple discrimination between samples with or without PrP^{Sc} is not very feasible.

To overcome this difficulty, samples are often treated with non-specific proteases such as Proteinase K (PK) or dispase before the denaturation and detection of PrP^{Sc}. (See, e.g., U.S. Patent No. 7,163,798 B2 and European Patent 1 119 773 B1.) Because of the structure of the PrP^{Sc} isoform, PrP^{Sc} is largely resistant to protease digestion. The PrP^{C} isoform, on the other hand, is completely degraded by treatment with such proteases. Thus, treatment of samples with a non-specific protease such as PK under some conditions (e.g., 50 µg/mL for 30 min at 37°C) can eliminate any PrP^{C} present and leave the protease-resistant core of PrP^{Sc} (amino acid residues from about 90 to about 231) generally intact.

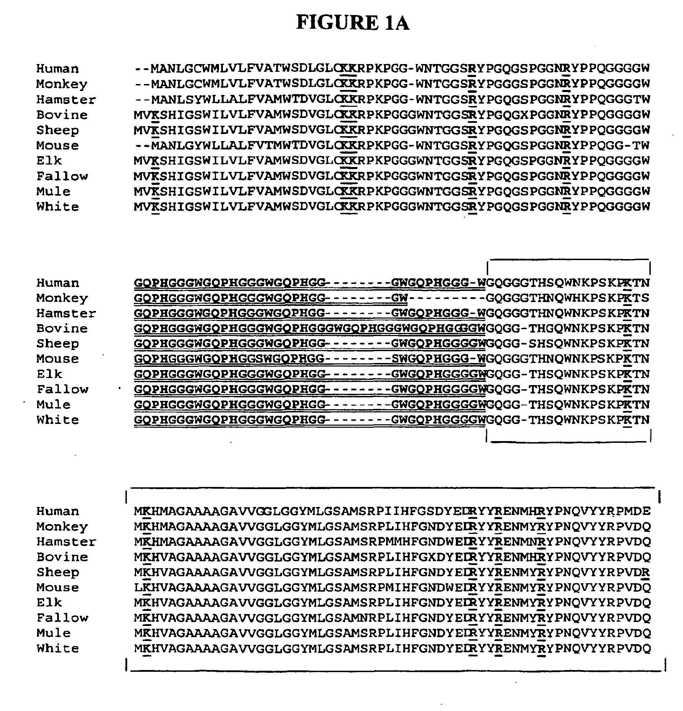

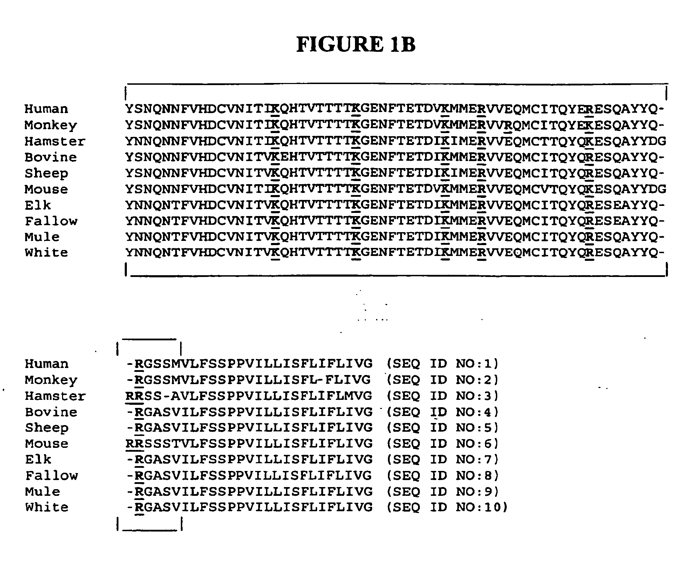

One major limitation of PK treatment is the degradation of the amino-terminal, or amino-proximal residues (from about 23 to about 89) of PrP^{Sc}. This region of PrP can be useful for immunodetection since it contains a number of epitopes including epitopes in the octarepeat region. The octarepeat region contains several copies of the octarepeat sequence GQPHGG(G/S)(-/G)W (SEQ ID NO: 11). This octarepeat sequence is highly conserved in prion proteins from different species and it repeats four times between residues about 58-89, numbered according to human or Syrian hamster PrP full-length sequence, in most species except bovine PrP that has 5 repeats and monkey PrP that has 3 repeats. (See Figure 1 for an alignment of PrP sequences of several species.) This sequence is unique to PrP and several PrP-specific antibodies have been shown to bind this sequence, for example, POM2, POM11, POM12, POM14, 3B5, 4F2, 13F10, SAF-15, SAF-31, SAF-32, SAF-33, SAF-34, SAF-35 and SAF-37. (See, e.g., Polymenidou et al. (2005) Lancet Neurol. 4:805-814; Krasemann et al. (1996) Mol. Medicine 2:725-734; Féraudet, et al. (2005) J. Biol. Chem. 280:11247-11258; U.S. Patent No. 7,097,997 B1.)

In general, most antibodies bind an epitope that is only presented once on a protein, even though antibodies inherently contain two binding sites (i.e. two binding arms). This type of binding is termed monovalent binding. Monovalent binding is weaker than an event where both antibody-binding sites simultaneously interact with two epitopes on the same protein (i.e. multivalent binding). The overall strength of multivalent binding, called avidity, is greater than affinity, the strength of binding of a single site, since both binding sites must dissociate at the same time for the antibody to release the antigen. For example, this property is very important in the binding of antibodies to bacteria or viruses, which usually have multiple identical epitopes on their surfaces. Thus, the octarepeat region of PrP allows enhanced signal through both the uniqueness of multiple octarepeat epitopes and the multivalent binding. Since PK digests residues 23-89 of the full length mature PrP^{Sc} (amino acids 23-231), the repertoire of useful antibodies after a complete PK digestion is limited to those that bind sequences within the protease-resistant core (amino acids ∼90-231), and antibodies against the octarepeat region cannot be used. Therefore the immunoassays using PK digestion are not very sensitive.

An additional disadvantage of using non-specific proteases like PK is that PrP^{Sc} is only partially resistant; given high concentrations and enough time most or even all 90-231 residues will be digested as well. It has been shown that some conformers of PrP^{Sc} are more sensitive than others to digestion and such treatment reduces the sensitivity of detection. (See, e.g., Safar et al. (1998) Nat. Med. 4:1157-65). Consistent with this is the finding that PK reduces levels of PrP^{Sc} and prion infectivity by several logs (See McKinley et al. (1983) Cell 35:57-62).

A recently described immunoassay method addressed the limitation discussed above by controlling PK digestion conditions so that PrP^{C} is completely digested but PrP^{Sc} is only partially digested and all or some of the octarepeat region is retained. (See U.S. Patent No. 7,097,997 B1.) However, this solution is not ideal as the appropriate condition may vary for different samples and therefore may be laborious to achieve and hard to standardize.

Thus, there remains a need for a specific, sensitive and relatively simple or quick method and a kit using such method to detect the presence of the pathogenic prion proteins in various samples, for example in samples obtained from living subjects, in blood supplies, in farm animals and in other human and animal food supplies. This invention is directed to this, as well as other, important ends.

### SUMMARY OF THE INVENTION

The present disclosure provides a new, simple, specific, and sensitive way, in the forms of assay methods and kit as defined in the claims, to detect the presence of pathogenic prion proteins, which may be used, *inter alia,* in connection with methods for diagnosing a prion-related disease (e.g., in human or non-human animal subjects), for ensuring a substantially PrP^{Sc}-free blood supply, blood products supply, or food supply, for analyzing organ and tissue samples for transplantation, for monitoring the decontamination of surgical tools and equipment, as well as any other situation in which knowledge of the presence or absence of the pathogenic prion is important.

The assay methods in the present disclosure take advantage of a treatment with a site-specific protease that does not cleave in the octarepeat region of known PrP sequences or in the protease-resistant core of PrP^{Sc} after a substantially complete digestion, while cleaving away at least part of the amino acids in PrP^{C} that correspond to the protease-resistant core in PrP^{Sc}, which makes it possible and convenient for epitopes in the octarepeat region or even those further upstream to be utilized in combination with epitopes in the protease-resistant core region for a specific and sensitive detection of PrP^{Sc}.

The present invention relates, in part, to a method for detecting the presence of PrP^{Sc} in a sample by contacting the sample with a site-specific protease wherein said site-specific protease is trypsin or staphylococcus aureus V8 protease (S-V8) under conditions in which proteolytic digestion of prion proteins is substantially complete, preventing further protease digestion of the sample, and detecting the presence of any PrP^{Sc} by binding to at least two binding partners, which include but are not limited to antibodies and aptamers, wherein one binding partner specifically binds an epitope in the amino-proximal region of PrP, preferably in the octarepeat region, and another binding partner specifically binds an epitopes within the protease-resistant core region.

The invention provides a method for detecting the presence of a pathogenic form of prion protein, or PrP^{Sc}, using at least one site-specific protease, wherein said at least one site-specific protease is trypsin or *Staphylococcus aureus* V8 protease (S-V8), for a substantially complete digestion of the proteins in a sample that is suspected of containing the PrP^{Sc}, wherein the sample may or may not contain a normal form of prion protein (PrP^{C}). The PrP^{Sc} has no cleavage site for the site-specific protease within the octarepeat region or between the octarepeat region and the protease-resistant core. Therefore, a fragment of amino-proximal region including the octarepeat region remains connected to the protease-resistant core of the PrP^{Sc}, which protease-resistant core remains intact, after the substantially complete proteolytic digestion. The PrP^{C}, if present, has at least one available cleavage site for the site-specific protease within amino acid region corresponding to the protease-resistant core of PrP^{Sc}, and the at least one available cleavage site is cleaved by the substantially complete proteolytic digestion.

The method further comprises a step of preventing any further proteolytic digestion following the substantially complete proteolytic digestion, which can be accomplished by adding a protease inhibitor or by removing the site-specific protease.

The method further comprises a step of denaturing the site-specific-protease-treated PrP^{Sc} after the step of preventing further proteolytic digestion, thereby providing denatured PrP^{Sc}.

The method further comprises a step of detecting the presence of PrP^{Sc}, using at least two binding partners, a first binding partner and a second binding partner, which include but are not limited to antibodies and aptamers, wherein the first binding partner specifically binds a first epitope within the fragment of amino-proximal region of the PrP^{Sc} that remains connected to the protease-resistant core after the substantially complete proteolytic digestion, preferably in the octarepeat region, and the second binding partner specifically binds a second epitope within the protease-resistant core region of the PrP^{Sc}, wherein the second epitope is in a region of the PrP^{C} that is separated from the first epitope after the substantially complete proteolytic digestion.

In certain embodiments, the PrP^{Sc} comprises a sequence selected from SEQ ID NOs. 1 to 10.

In a preferred embodiment, the first binding partner binds to an epitope that is within the octarepeat region. Exemplary first binding partners in these embodiments include monoclonal antibodies such as POM2, POM 11, POM12, POM14, 3B5, 4F2, 13F10, SAF-15, SAF-31, SAF-32, SAF-33, SAF-34, SAF-35, and SAF-37.

In certain other embodiments, the first binding partner binds to an epitope that is outside of the octarepeat region. Exemplary first binding partners in these embodiments include monoclonal antibodies such as BAR210, BAR231, and 14D3.

In certain embodiments, the site-specific protease used in the method is trypsin, which specifically hydrolyzes peptide bonds at the carboxyl side of Lysine (K) and Arginine (R) residues unless the amino acid on the carboxyl side of K and R is Proline (P). For these embodiments, the second binding partner can be specific to any epitope that is within the protease-resistant core of PrP^{Sc} and that has been cleaved away and thus is separated from the first epitope in PrP^{C} by trypsin digestion. Exemplary second binding partners in these embodiments include monoclonal antibodies such as 3F4, POM1, POM4, POM5, POM6, POM7, POM8, POM9, POM10, POM 13, POM 15, POM 16, POM 17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4, and BDI 115.

In a preferred embodiment of the method in which trypsin is used, the second binding partner specifically binds to an epitope located within the globular domain of PrP^{C} (i.e., from about amino acid 122 to about amino acid 231 of PrP^{C}). Exemplary second binding partners in these embodiments include monoclonal antibodies such as POM1, POM4, POM5, POM6, POM7, POM8, POM9, POM10, POM13, POM15, POM 16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, and BDI115.

In certain other embodiments, the site-specific protease used in the method is S-V8 which specifically cleaves on the carboxyl side of Glutamic Acid (E) and Aspartic Acid (D) residues. For these embodiments, the second binding partner can be specific to any epitope that is within the protease-resistant core of PrP^{Sc} and that has been cleaved away in PrP^{C} by S-V8. Exemplary second binding partners in these embodiments include monoclonal antibodies such as SAF-53, SAF-54, SAF-60, SAF-61, SAF-66, SAF-69, SAF-70, SAF-75, SAF-76, Pri917, BAR234, Sha31, 11B9, 12F10, 6H4, and POM5.

In certain embodiments of the method, the detecting step is carried out using an ELISA, preferably a Sandwich ELISA. One such embodiment utilizes a first binding partner which binds an epitope within the amino-proximal region of PrP, particularly within the octarepeat region, as a capture binding partner and a second binding partner which binds an epitope within the protease-resistant core as a detection binding partner. In another such embodiment, the second binding partner which binds an epitope within the protease-resistant core is utilized as a capture binding partner and the first binding partner which binds an epitope within the amino-proximal region is utilized as a detection binding partner.

In certain preferred embodiments, the detection binding partners discussed above are labeled. In certain embodiments among these, the detection binding partners are labeled with an alkaline phosphatase (AP) conjugate.

In certain embodiments, the binding partners used in the method are antibodies, preferably monoclonal antibodies, specific to the first epitope and the second epitope, wherein the antibodies can be selected from, but are not limited to, the exemplary monoclonal antibodies disclosed herein.

In certain other embodiments, the binding partners used in the method are aptamers specific to the first epitope and the second epitope.

In one embodiment of the method, said site-specific protease is immobilized on a solid support.

In another embodiment, the protease inhibitor used in the method to prevent further proteolytic digestion of the sample by the site-specific protease following the substantially complete proteolytic digestion is phenylmethylsulphonyl fluoride (PMSF).

In certain embodiments, the denaturing step in the method comprises treating the Digested samples with a guanidinium compound, e.g., guanidine hydrochloride (GdnHCl) or guanidine thiocyanate (GdnSCN).

In certain embodiments among the ones that used a guanidinium compound as the denaturant, a further step of diluting the samples is involved after denaturation.

In certain other embodiments, the denaturing step in the method comprises treating the digested samples with high pH or low BH and, optionally, neutralizing said high pH or said low pH after said denaturing.

In certain embodiments of the method, the samples are obtained from a living or once-living organism. Exemplary organisms in these embodiments include human, monkey, hamster, bovine, sheep, mouse, elk, and deer.

In certain embodiments, the sample used in the method is derived from the group consisting of food supply, whole blood, blood products, blood fractions, blood components, plasma, platelets, serum, cerebrospinal fluid, organs, cells, brain tissue, nervous system tissue, muscle tissue, fatty tissue, bone marrow, urine, tears, non-nervous system tissue, biopsies, necropsies, and contaminated instruments.

In certain embodiments, the sample used in the method is derived from the group consisting of whole blood, blood products, blood fractions, blood components, plasma, platelets, red blood cells, and serum.

In certain embodiments, the sample is obtained from a biopsy, autopsy or necropsy.

The present invention relates also, in part, to a kit for detecting the presence of a pathogenic form of prion protein, or PrP^{Sc}, in a sample suspected of containing the PrP^{Sc}, wherein the sample may or may not contain a normal form of prion protein (PrP^{C}), the kit comprising reagents that are useful in the method discussed herein.

Specifically, the kit comprises at least one site-specific protease wherein said at least one site-specific protease is trypsin or S-V8, wherein the PrP^{Sc} has no cleavage site for the site-specific protease within the octarepeat region or between the octarepeat region and the protease-resistant core, therefore a fragment of amino-proximal region of the PrP^{Sc} including the octarepeat region remains connected to the protease-resistant core after a substantially complete proteolytic digestion of PrP^{Sc} by the protease. The PrP^{C}, if present, has at least one available cleavage site for the site-specific protease within amino acid region corresponding to the protease-resistant core of the PrP^{Sc}.

The kit further comprises at least two binding partners, a first binding partner and a second binding partner, which include but are not limited to antibodies and aptamers. The first binding partner specifically binds a first epitope which is located within the fragment of amino-proximal region that remains connected to the protease-resistant core after a substantially complete proteolytic digestion of the PrP^{Sc} by the site-specific protease in the kit. The second binding partner specifically binds a second epitope which is located within the protease-resistant core, wherein the second epitope is in a region of the PrP^{C} that is separated from the first epitope after a substantially complete proteolytic digestion of the PrP^{C} by the site-specific protease in the kit.

The kit further comprises an instruction for using the kit to detect the presence of any pathogenic form of prion protein.

Optionally, the kit further comprises a protease inhibitor which is capable of inhibiting the activity of the site-specific protease in the kit.

Again optionally, the kit further comprises a denaturant which is capable of denaturing the PrP^{Sc}.

In certain embodiments of the kit, the PrP^{Sc} comprises a sequence selected from SEQ ID NOs. 1 to 10.

In certain, preferred, embodiments of the kit, the first binding partners specifically binds an epitope within the octarepeat region. Exemplary first binding partners in these embodiments include monoclonal antibodies such as POM2, POM11, POM12, POM14, 3B5, 4F2, 13F10, SAF-15, SAF-31, SAF-32, SAF-33, SAF-34, SAF-35, and SAF-37.

In certain other embodiments of the kit, the first binding partner specifically binds an epitope that is outside of the octarepeat region. Exemplary first binding partners in these embodiments include monoclonal antibodies such as BAR210, BAR231, and 14D3.

In certain embodiments of the kit, the site-specific protease in the kit is trypsin. For these embodiments, exemplary second binding partners in these embodiments include monoclonal antibodies such as 3F4, POM1, POM4, POM5, POM6, POM7, POM8, POM9, POM 10, POM 13, POM15, POM 16, POM 17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4, and BDI115.

In a preferred embodiment of the kit in which trypsin is the site-specific protease, the second binding partner specifically binds to an epitope located within the globular domain of PrP^{C} (i.e., from about amino acid 122 to about amino acid 231 of PrP^{C}). Exemplary second binding partners in these embodiments include monoclonal antibodies such as POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, and BDI11 15.

In certain other embodiments, the site-specific protease in the kit is S-V8. For these embodiments, exemplary second binding partners in these embodiments include monoclonal antibodies such as SAF-53, SAF-54, SAF-60, SAF-61, SAF-66, SAF-69, SAF-70, SAF-75, SAF-76, Pri917, BAR234, Sha31, 11B9, 12F10, 6H4, and POM5.

In certain embodiments of the kit, the kit comprises an ELISA kit, preferably a Sandwich ELISA kit. Among these embodiments, certain embodiments have the first binding partner as a capture binding partner and the second binding partner as a detection binding partner. Yet certain other embodiments have the second binding partner as a capture binding partner and the first binding partner as a detection binding partner.

In certain embodiments of the kit, the detection binding partners discussed above are labeled. In certain embodiments among these, the detection binding partners are labeled with an alkaline phosphatase (AP) conjugate.

In certain embodiments, the binding partners in the kit are antibodies, preferably monoclonal antibodies, specific to the first epitope and the second epitope, wherein the antibodies can be selected from, but are not limited to, the exemplary monoclonal antibodies shown above.

In certain other embodiments, the binding partners in the kit are aptamers specific to the first epitope and the second epitope.

In one embodiment of the kit, the site-specific protease is immobilized.

In another embodiment of the kit, the optional protease inhibitor is phenylmethylsulphonyl fluoride (PMSF).

In certain embodiments of the kit, the optional denaturant is a guanidinium compound.

In certain other embodiments of the kit, the optional denaturant is a basic reagent or an acidic reagent.

### BRIEF DESCRIPTION OF THE DRAWINGS

FIG. 1A and FIG. 1B depict an amino acid sequence alignment and trypsin cleavage sites of prion proteins from human (SEQ ID NO:1), monkey (SEQ ID NO:2), Syrian hamster (hamster) (SEQ ID NO:3), bovine (SEQ ID NO:4), sheep (SEQ ID NO:5), mouse (SEQ ID NO:6), elk (SEQ ID NO:7), fallow deer (fallow) (SEQ ID NO:8), mule deer (mule) (SEQ ID NO:9), and white-tailed deer (white) (SEQ ID NO:10). The octarepeat regions are double-underlined. The protease-resistant core regions are indicated with brackets above and below the sequence alignment. (It should be noted that the exact beginning and ending amino acid locations of the protease-resistant core regions vary slightly among different species and different strains and with different protease digestion conditions.) The trypsin cleavage sites, amino acids Lysine (K) and Arginine (R) that do not have Proline (P) on their carboxyl sides, are single-underlined.

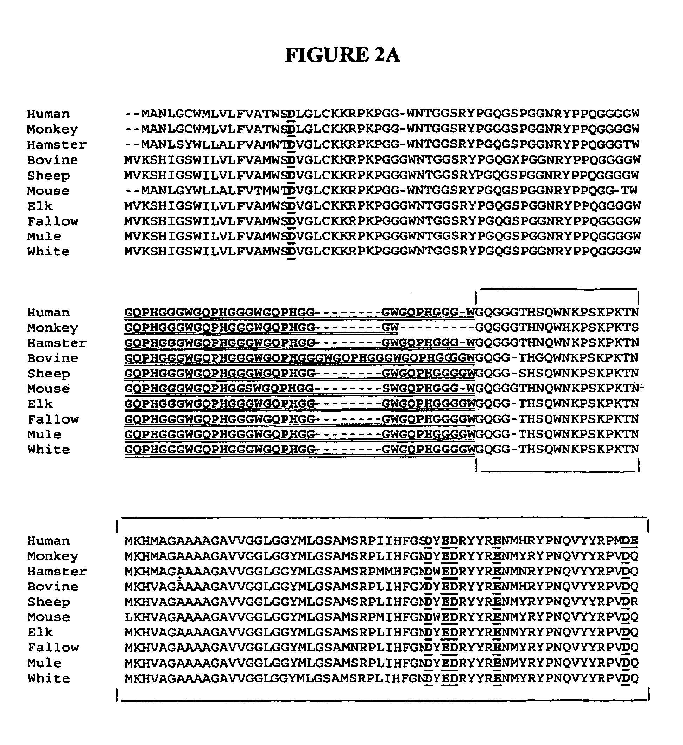

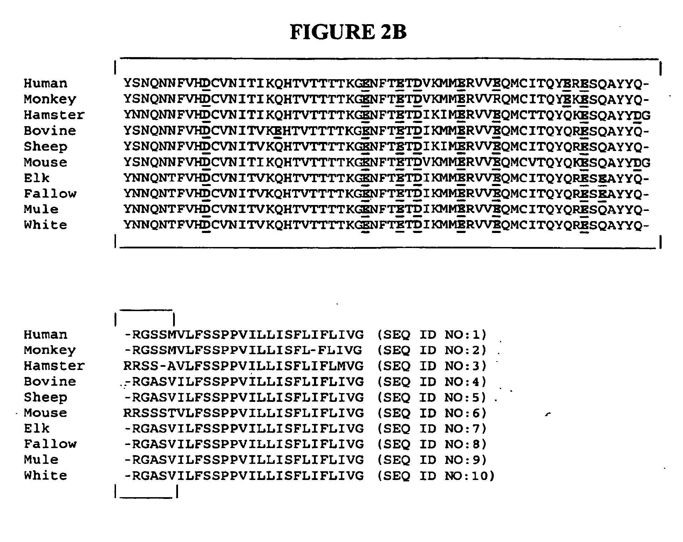

FIG. 2A and FIG. 2B depict an amino acid sequence alignment and S-V8 cleavage sites of prion proteins from human (SEQ ID NO: 1), monkey (SEQ ID NO:2), Syrian hamster (hamster) (SEQ ID NO:3), bovine (SEQ ID NO:4), sheep (SEQ ID NO:5), mouse (SEQ ID NO:6), elk (SEQ ID NO:7), fallow deer (fallow) (SEQ ID NO:8), mule deer (mule) (SEQ ID NO:9), and white-tailed deer (white) (SEQ ID NO:10). The octarepeat regions are double-underlined. The protease-resistant core regions are indicated with brackets above and below the sequence alignment. The S-V8 cleavage sites, amino acids Aspartic acid (D) and Glutamic acid (E), are single-underlined.

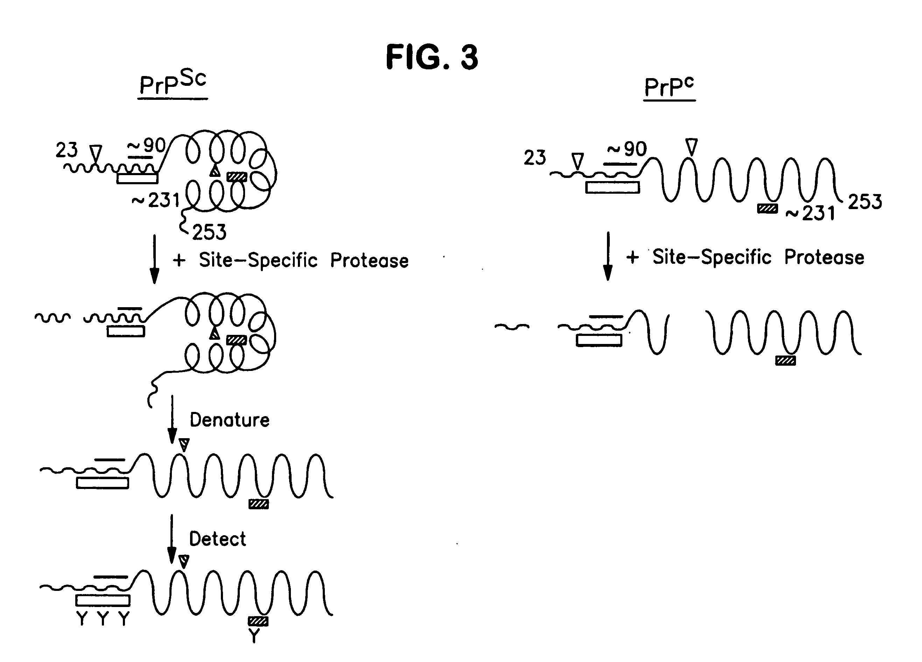

FIG. 3 illustrates the scheme of one embodiment of the invention. The single line represents the prion protein, the coiled section represents the protease-resistant core of the PrP^{Sc}, and the wavy section represents the more alpha-helical sections of the PrP^{C} and PrP^{Sc} isoforms. The numbers by various locations of PrP^{Sc} or PrP^{C} protein represent the amino acid boundaries of different regions. The boxes indicate the epitope regions recognized by the anti-prion antibodies, the solid bars represent the octarepeat region, and the triangles represent the site-specific protease cleavage sites. Open boxes represent epitope regions within the amino-proximal region, preferably within the octarepeat region, while hatched boxes represent epitope regions within the protease-resistant core of PrP^{Sc} or the same epitope regions within the amino acid sequences of PrP^{C} that correspond to the protease-resistant core. Open triangles represent available cleavage sites for the site-specific protease in the native form of PrP^{Sc} or PrP^{C}. Hatched triangles represent unavailable cleavage sites for the site-specific protease in the native form of PrP^{Sc}. The Y's represent antibodies.

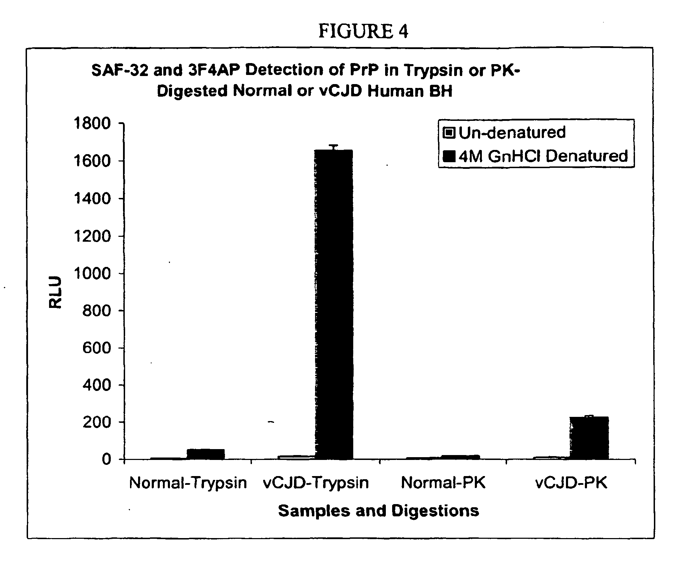

FIG. 4 illustrates the detection, by Sandwich-ELISA, of PrP in normal or vCJD human brain homogenate (BH) samples digested with trypsin or PK, using SAF-32 as the capture antibody and alkaline phosphatase-conjugated 3F4 (3F4AP) as the detection antibody. Details of the experiment are disclosed in Example 6. After protease digestion, samples were either left un-denatured or denatured with 4 M GdnHCl before detection of PrP^{Sc}. Relative amounts of detected signals are shown in relative light units (RLU).

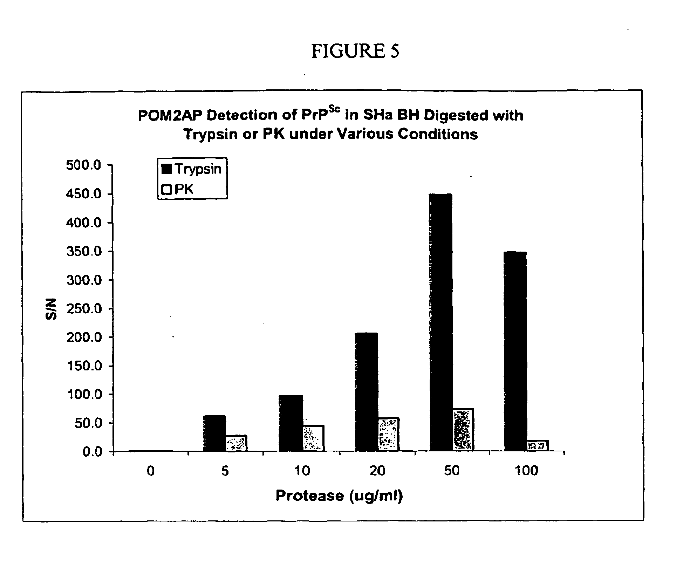

FIG. 5 illustrates the detection, by Sandwich-ELISA, of PrP^{Sc}, in scrapie-infected Syrian hamster (SHa) BH samples digested with trypsin or PK under various conditions, using 3F4 as the capture antibody and alkaline phosphatase-conjugated POM2 (POM2AP) as the detection antibody. Details of the experiment are disclosed in Example 7. Ratios of scrapie-infected signals over normal BH signals are shown (S/N values).

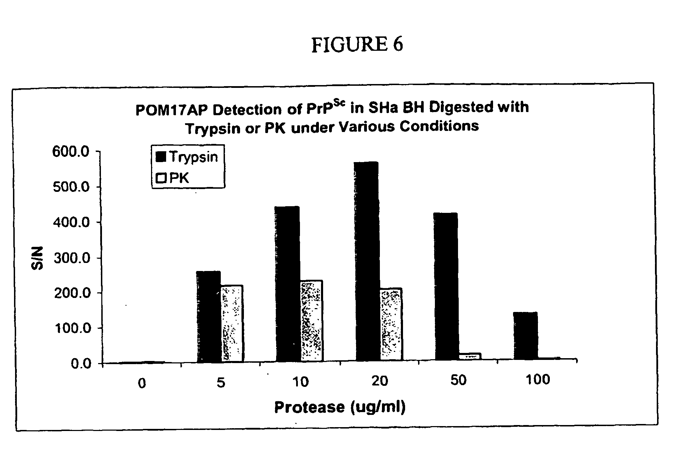

FIG. 6 illustrates the detection, by Sandwich-ELISA, of PrP^{Sc} in scrapie-infected SHa BH samples digested with trypsin or PK under various conditions, using 3F4 as the capture antibody and alkaline phosphatase-conjugated POM 17 (POM 17AP) as the detection antibody. Details of the experiment are disclosed in Example 7. Ratios of scrapie-infected signals over normal BH signals are shown (S/N values).

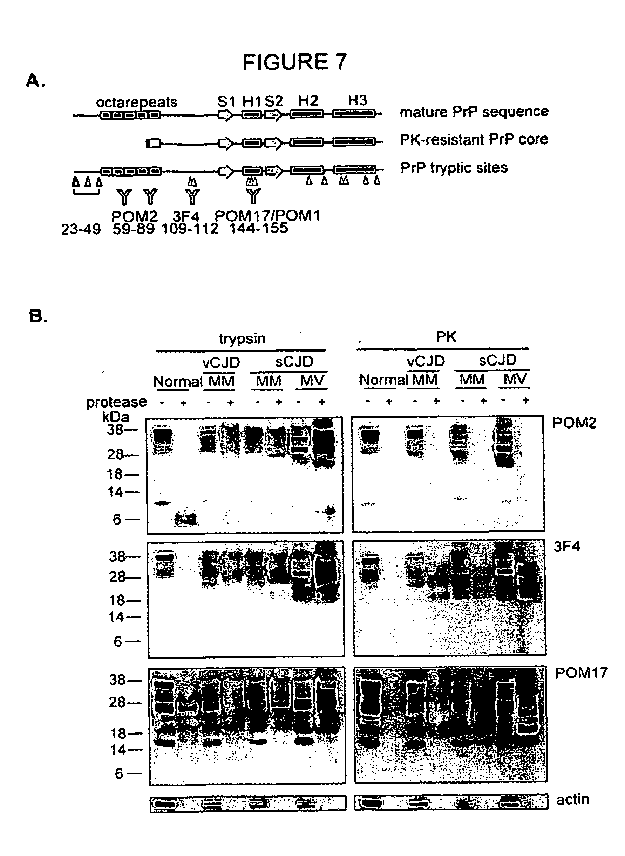

FIG. 7A illustrates a schematic of the full-length mature PrP sequence, the PK-resistant core of PrP^{Sc}, and the trypsin cleavage site map. Trypsin cleavage sites are indicated by triangles, with the sites lying within the PK-resistant core shown as gray triangles to indicate that they are generally not available for trypsin cleavage in PrP^{Sc}. FIG. 7B illustrates the result of a Western blot analysis. Details of the experiment are disclosed in Example 8. Fifty µg/ml trypsin or PK digestions were compared for normal, vCJD MM (codon 129 polymorphism, the white vCJD strain), sCJD MM, and sCJD MV BH samples. Samples were separated by SDS-PAGE and immunoblotted with POM2, 3F4, or POM17 antibodies. An actin immunoblot was performed as a loading control.

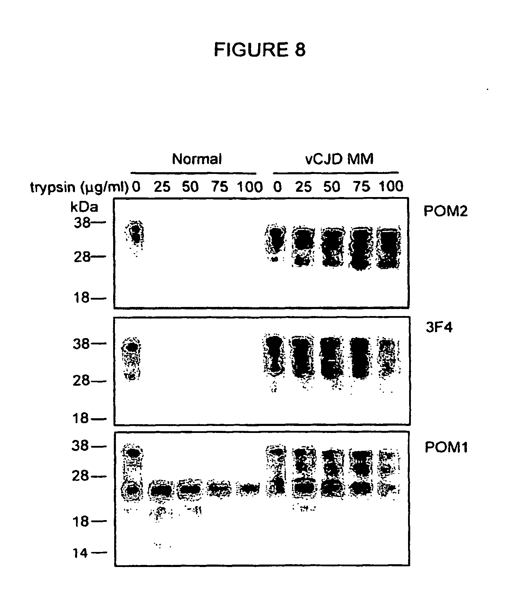

FIG. 8 illustrates the result of another Western blot analysis. Details of the experiment are disclosed in Example 8. Normal or a vCJD BH sample was digested with 0, 25, 50, 75 or 100 µg/ml trypsin and then analyzed by immunoblotting with POM2, 3F4, or POM 1 antibodies.

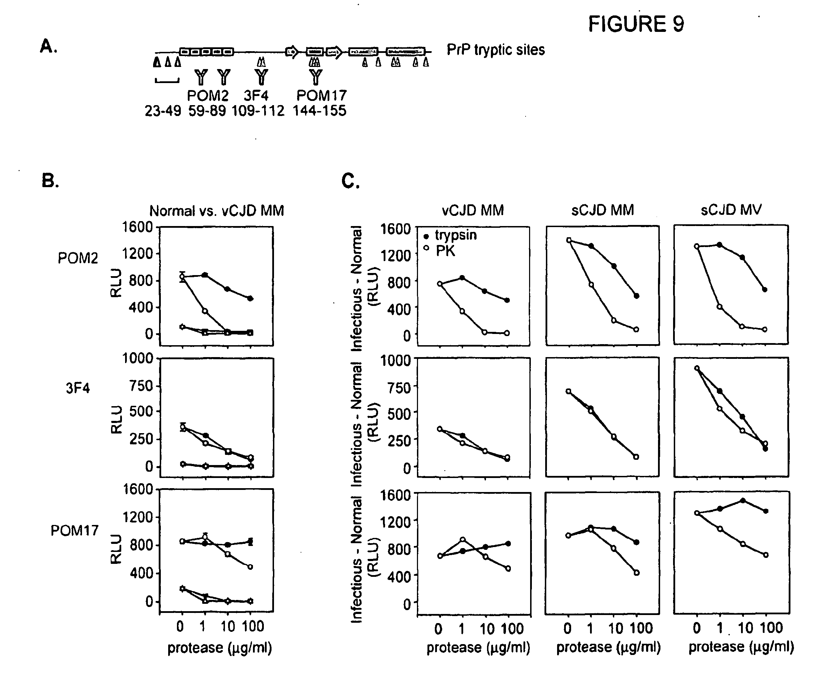

FIG. 9A illustrates a schematic of the full-length mature PrP sequence with the trypsin cleavage sites and the locations of several antibody epitopes marked. The trypsin cleavage sites at the 3F4 and the POM17 epitope regions are shown as gray triangles, indicating that they are generally not available for trypsin cleavage in PrP^{Sc}. FIG. 9B and FIG. 9C illustrate the result of a "passive coating" direct ELISA experiment. Details of the experiment are disclosed in Example 9. Normal or CJD BHs were digested with increasing concentrations of trypsin (filled symbols) or PK (open symbols). Digests were centrifuged and PrP^{Sc} pellets were denatured and detected by direct ELISA with POM2 (top panels), 3F4 (middle panels), or POM 17 (bottom panels) antibodies. Relative amounts of detected signals are shown in relative light units (RLU). FIG. 9B illustrates the raw data of the normal BH (triangles) and the vCJD MM BH (circles). FIG. 9C illustrates the normalized data of the vCJD MM, sCJD MM, and sCJD MV BH samples. Results are expressed in "Infectious - Normal (RLU)".

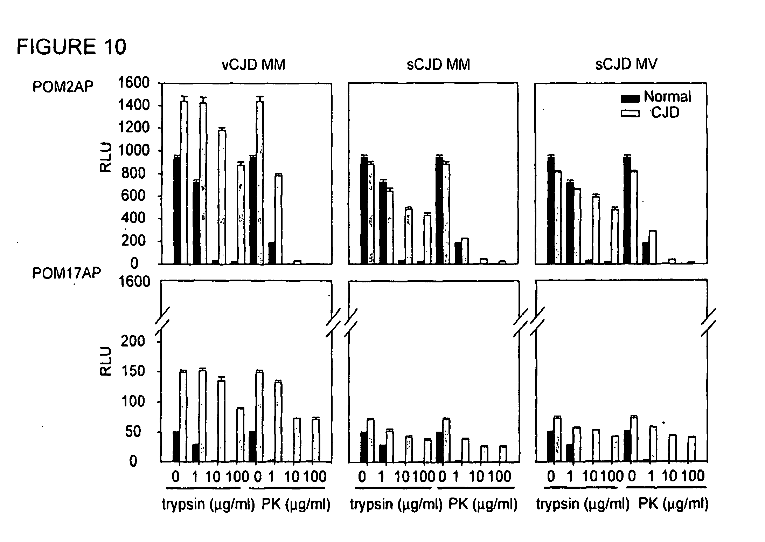

FIG. 10 illustrates, in bar graphs, the detection, by Sandwich-ELISA, of PrP in normal or various CJD human BH samples digested with increasing concentrations of trypsin (the left half of each panel) or PK (the right half of each panel), using 3F4 as the capture antibody and POM2AP or POM17AP as the detection antibody. Details of the experiment are disclosed in Example 10. Relative amounts of detected signals obtained as raw data are shown in relative light units (RLU). The solid black bars represent data of the normal BH, while the gray bars represent data of various CJD BHs.

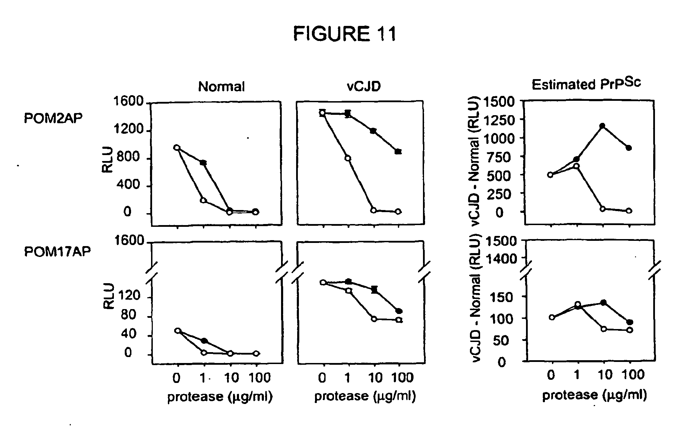

FIG. 11 illustrates, in line graphs, the detection, by Sandwich-ELISA, of PrP in normal or vCJD human BH sample digested with increasing concentrations of trypsin (closed circles) or PK (open circles), using 3F4 as the capture antibody and POM2AP or POM17AP as the detection antibody. Details of the experiment, which is a part of the experiment of FIG. 10, are disclosed in Example 10. Relative amounts of detected signals obtained as raw data are shown in relative light units (RLU) in the left and the middle panels. The amounts of signals contributed by PrP^{Sc} were estimated by subtracting Normal BH-derived signals from vCJD BH-derived signals and shown in the right panels as "vCJD - Normal (RLU)".

### DETAILED DESCRIPTION

The practice of the present disclosure will employ, unless otherwise indicated, conventional methods of chemistry, biochemistry, molecular biology, immunology and pharmacology, within the skill of the art. Such techniques are explained fully in the literature. See, e.g.; Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); Peters and Dalrymple, Fields Virology (2d ed), Fields et al. (eds.), B.N. Raven Press, New York, NY.

### Definitions

The following select terms will be discussed in the context used herein. Both the plural and singular forms of a term are included regardless of the form discussed.

"Prion," "prion protein," "PrP protein," and "PrP" are used interchangeably to refer to both the pathogenic prion protein form (also referred to as scrapie protein, pathogenic protein form, pathogenic isoform, pathogenic prion and PrP^{Sc}) and the non-pathogenic prion form (also referred to as the normal form, cellular protein form, cellular isoform, nonpathogenic isoform, nonpathogenic prion protein and PrP^{C}), as well as the denatured form and various recombinant forms of the prion protein that may not have either the pathogenic conformation or the normal cellular conformation.

Use of the terms "prion," "prion protein," "PrP protein," "PrP" or "conformational disease protein" is not meant to be limited to polypeptides having the exact sequences to those described herein. It is readily apparent that the terms encompass conformational disease proteins from any of the identified or unidentified species (e.g., human, bovine) or diseases (e.g., Alzheimer's, Parkinson's, etc.). See also, co-owned U.S. Patent Publications 20050118645 and 20060035242 and PCT Publication WO 06/076687. One of ordinary skill in the art in view of the teachings of the present disclosure and the art can determine regions corresponding to the sequences disclosed herein in any other prion proteins, using for example, sequence comparison programs (e.g., Basic Local Alignment Search Tool (BLAST)) or identification and alignment of structural features or motifs.

"Pathogenic" means that the protein actually causes the disease, or the protein is associated with the disease and, therefore, is present when the disease is present. Thus, a pathogenic protein, as used herein, is not necessarily a protein that is the specific causative agent of a disease. A "pathogenic form" of a protein means a conformation of a protein that is present when the disease is present, but it may or may not be infectious. An example of a pathogenic conformational disease protein, or a pathogenic form of a protein, is PrP^{Sc}. Accordingly, the term "non-pathogenic" or "normal form" describes a protein that does not normally cause disease or is not normally associated with causing disease. An example of a non-pathogenic or a normal form of conformational disease protein is PrP^{C}.

"Prion-related disease" refers to a disease caused in whole or in part by a pathogenic prion protein, or a pathogenic form of prion protein (e.g., PrP^{Sc}). Examples of prion-related disease include, without limitation, scrapie, bovine spongiform encephalopathies (BSE), mad cow disease, feline spongiform encephalopathies, kuru, Creutzfeldt-Jakob Disease (CJD), new variant Creutzfeldt-Jakob Disease (nvCJD), chronic wasting disease (CWD), Gerstmann-Strassler-Scheinker Disease (GSS), and fatal familial insomnia (FFI).

The term "denature" or "denatured" has the conventional meaning as applied to protein structure and means that the protein has lost its native secondary and tertiary structure. With respect to the pathogenic prion protein, a "denatured" pathogenic prion protein no longer retains the native pathogenic conformation and thus the protein is no longer "pathogenic" The denatured pathogenic prion protein has a conformation similar or identical to the denatured non-pathogenic prion protein.

The terms "label," "labeled," "detectable label," and "detectably labeled" refer to a molecule capable of detection, including, but not limited to, radioactive isotopes, fluorescers, luminescers, chemiluminescers, enzymes, enzyme substrates, enzyme cofactors, enzyme inhibitors, chromophores, dyes, metal ions, metal sols, ligands (e.g., biotin or haptens), fluorescent nanoparticles, gold nanoparticles, and the like. Particular examples of labels that can be used include, but are not limited to fluorescein, rhodamine, dansyl, umbelliferone, Texas red, luminol, acridinium esters, NADPH, beta-galactosidase, horseradish peroxidase, glucose oxidase, alkaline phosphatase and urease. The label can also be an epitope tag (e.g., a His-His tag), an antibody or an amplifiable or otherwise detectable oligonucleotide.

"Proteolytic digestion" or "protease digestion" refers to the directed digestion or degradation of a protein by a protease, through hydrolysis, or cleavage, of peptide bonds in the protein.

A "site-specific protease" refers to an enzyme that cleaves peptide bonds (a protease) at one type or a small number of different amino acid residues in a protein substrate. "Cleavage sites" for a site-specific protease refer to the specific amino acid residues in a protein substrate at which the adjacent peptide bonds are hydrolyzed, or "cleaved", by the site-specific protease under normal proteolytic digestion conditions. The site-specific protease is distinguished from the non-specific proteases like proteinase K (which cleaves at aliphatic, aromatic and hydrophobic residues) and carboxypeptidase Y (which cleaves all residues sequentially beginning at the carboxy terminal). For example, trypsin is a site-specific protease that cleaves only at Lys and Arg residues, especially when the amino acid on the carboxyl side of Lys and Arg is not Pro. (See, e.g., Neurath and Schwert (1950) Chem. Rev. 46:70; Walsh and Neurath (1964) Proc.Natl. Acad Sci. USA 52:884-889; Walsh (1970) Meth. Enzymol. 19:41.) Therefore, the cleavage sites for trypsin mean Lys and Arg residues when the amino acid on the carboxyl side of Lys and Arg is not Pro. The cleavage sites for trypsin are also referred to as "tryptic sites". Another example is the protease S-V8, which specifically cleaves at Glu and Asp residues (See, e.g., Drapeau et al. (1972) J. Biol. Chem. 247:6720-6726 and Houmard and Drapeau (1972) Proc. Natl. Acad. Sci. USA 69:3506-3509). Trypsin and S-V8 are commercially available from companies such as Pierce, part of Thermo Fisher Scientific Inc., or Sigma-Aldrich, Inc.

A "substantially complete" digestion or "substantially digested" means a digestion in which a protein has been cleaved by a protease in at least 90%, preferably 99%, of all available protease cleavage sites. By "available cleavage site" is intended a site having the amino acid sequence recognized as the cleavage site by the protease and that is available for contact with the protease in the conformation of the protein. As an example, protease cleavage sites that occur within the protease-resistant core of the prion protein are generally not available to protease digestion when the prion protein is in the PrP^{Sc} conformation, and thus are not "available cleavage sites" in PrP^{Sc}.

The "octarepeat region" refers to a repeated sequence region that is found close to the N-terminal of prion proteins from all species so far identified. The octarepeat generally contains between 3 and 5, usually 4, copies of an 8 (or 9) amino acid sequence usually written as GQPHGG(G/S)(-/G)W (SEQ ID NO: 11). This sequence is highly conserved (although this sequence may vary slightly in some of the repeats) and generally occurs within about residues 58-89, numbered according to human and hamster PrP species. The octarepeat region is usually adjacent, and N-terminal proximal, to the protease-resistant region.

The "protease-resistant core" of the prion protein (sometimes called the "proteinase K resistant core") is defined by the region of the prion protein in the PrP^{Sc} conformation that remains after exposure of the PrP^{Sc} to proteinase K under condition that are sufficient to substantially digest the prion protein in the PrP^{C} form. In general, for most species of prion protein, the protease-resistant core region includes the regions from about amino acid 90 to about amino acid 231 numbered according to human and hamster PrP species. Figures 1A, 1B, 2A and 2B show an alignment of a number of prion proteins from different species where the bracketed region indicates the protease-resistant core region.

The "amino acid region of PrP^{C} corresponding to protease-resistant core" refers to the region of a PrP^{C} that is the same as the region that forms the protease-resistant core in the pathogenic form of the prion protein with the same primary amino acid sequence of said PrP^{C}. In general, for most species of prion protein, this region also refers to the region from about amino acid 90 to about amino acid 231 numbered according to human and hamster PrP species.

The "globular domain" of PrP^{C} is approximately from amino acid 122 to amino acid 231 numbered according to human PrP species.

By "epitope" herein is meant the region of a target protein which is recognized by (e. g. specifically binds to) binding partners which include without limitation antibodies and aptamers. An epitope can comprise 3 or more amino acids in a spatial conformation unique to the epitope. Generally, an epitope consists of at least 5 such amino acids and, more usually, consists of at least 8-10 such amino acids. Methods of determining spatial conformation of amino acids are known in the art and include, for example, x-ray crystallography and 2-dimensional nuclear magnetic resonance. Furthermore, the identification of epitopes in a given protein is readily accomplished using techniques well known in the art, such as by the use of hydrophobicity studies and by site-directed serology. See, also, Geysen et al., Proc. Natl. Acad. Sci. USA (1984) 81:3998-4002 (general method of rapidly synthesizing peptides to determine the location of immunogenic epitopes in a given antigen); U.S. Patent No. 4,708,871 (procedures for identifying and chemically synthesizing epitopes of antigens); and Geysen et al., Molecular Immunology (1986) 23:709-715 (technique for identifying peptides with high affinity for a given antibody).

"Amino-proximal region" refers to the N-terminal region of a mature PrP protein spanning from amino acid of 23 to about 89, numbered according to the human or hamster PrP sequence.

"Binding partner" refers to a molecule that can specifically recognize and bind, non-covalently, to a ligand such as an epitope. The binding partners as discussed in the current disclosure include without limitation antibodies and aptamers.

"Capture binding partner" refers to a binding partner that is coated on a solid support to specifically bind and capture a ligand, such as a PrP protein.

"Detection binding partner" refers to a binding partner that is added, in solution, to samples on a solid support to specifically detect any ligand that has been bound and captured by a capture binding partner.

In certain embodiments of the assay method, the binding partners are "aptamers." Aptamers are nucleic acids having the molecular recognition properties of antibodies. However, aptamers are smaller and less complex than antibodies, and thus maybe easier to manufacture and modify. (See, e.g., Osborne et al. (1997) Curr. Opin. Chem. Biol. 1:5-9; Bunka et al. (2006) Nat. Rev. Microbiol. 4:588-596.) Methods of generating aptamers specific to protein epitopes are readily available (see, e.g., Tuerk and Gold (1990) Science 249:505-510; Cox et al. (1998) Biotechnol Prog. 14:845-850; Cox et al. (2002) Nucleic Acids Res. 30:e108). Aptamers can be linked to enzymes that have an easily detectable activity (see, e.g., Drolet et al. (1996) Nat. Biotechnol. 14:1021).

"Treating" or "Treatment" of a sample with a protease means that the sample and the protease are brought into contact and stay together for sufficient length of time for a reaction, particularly hydrolysis or cleavage of peptide bonds, to occur.

### General Overview

Prior to the present disclosure, a published patent disclosed a method using PK to digest PrP^{Sc} and retain the octarepeat region for binding with a binding partner (a ligand), which requires a controlled digestion condition for partial digestion of PrP^{Sc} but complete digestion of PrP^{C}. (See U.S. Patent No. 7,097,997 B1.) One problem with this previously disclosed method is that the appropriate condition may vary for different samples, and therefore, may be laborious to achieve and difficult to standardize. This previously disclosed method did not involve any use of a second binding partner to bind the protease-resistant core of PrP^{Sc} in order to differentiate it from PrP^{C}.

To circumvent limitations associated with the use of non-specific proteases like PK, the current inventors investigated the use of other proteases, particularly site-specific proteases, that have no cleavage site within the octarepeat region of PrP. Therefore, even with a substantially complete digestion, the octarepeat region is not digested, making it consistently available for specific and strong binding by a binding partner. As compared to a partial digestion by PK, a substantially complete digestion by a site-specific protease is easier to carry out and standardize. In addition, the current disclosure includes the use of a second binding partner that binds to PrP^{Sc} but not to PrP^{C} due to the removal of the corresponding epitope from PrP^{C} by the protease, giving the currently disclosed method an advantage of high selectivity between the two forms of PrP.

Thus, the methods described herein relate to improvements that can increase specificity, sensitivity, ease of use and reproducibility of detection of PrP^{Sc} in a sample that may have a large amount of PrP^{C}.

The site-specific protease used is trypsin or S-V8, whose specific cleavages sites are not present in the octarepeat regions of the known PrP sequences as shown in Figures 1A, 1B, 2A and 2B. In addition, unlike the case with PK, digestion with trypsin or S-V8 does not affect prion infectivity, suggesting that PrP^{Sc} is more resistant to these proteases than to PK (See McKinley et al. (1983) Cell, Vol. 35, 57-62, and Langeveld et al. (2003) J. Infec. Dis. 188:1782-1789). Treatment of PrP^{Sc} with trypsin or S-V8 will result in protease resistant fragments with residues 49-231 or 23-231, respectively (numbered according to human or Syrian hamster species), due to the protection of the potential cleavage sites within the protease-resistant core of PrP^{Sc}. On the other hand, treatment of PrP^{Sc} with PK will result in multiple protease resistant fragments with N-terminus of PK-resistant fragments beginning around residues 80-100. The exact cleavage site of PrP^{Sc} by PK will vary since PrP^{Sc} can adopt different conformations (See Telling et al. (1996) Science 274:2079-2082). Therefore, treatment of PrP^{Sc} with trypsin or S-V8 will yield a more consistent, longer, and more useful fragment than treatment with PK.

Prior to the present disclosure, one publication suggested using trypsin to unmask an epitope to PrP^{Sc} and eliminate availability of a corresponding epitope of PrP^{C}. (See International Publication No. WO 99/19360 A1.) The immunoassay method suggested in WO 99/19360 A1 differs from the currently disclosed method, because the purpose or result of using trypsin in the currently disclosed method is not unmasking an epitope to PrP^{Sc}. In addition, the method suggested in WO 99/19360 A1 did not disclose binding PrP^{Sc} with an antibody specific for the octarepeat region. Therefore, the currently disclosed method offers an advantage of high sensitivity due to high avidity of binding partner's binding to the octarepeat region.

Another publication prior to the present disclosure, International Publication No. WO 03/00121 A1, disclosed a method for detecting the presence of prion proteins that involves a limited proteolysis by a protease such as PK. In one embodiment of the method described in WO 03/001211 A1, a protease such as trypsin was used briefly, prior to the steps of lysing the cells or tissues and treating the lysate with PK, and under a condition that will not disrupt the cell membrane, in order to facilitate tissue dissociation.

In addition, another publication prior to the present disclosure, International Publication No. WO 2006/088281 A1, disclosed a method for detecting a multimeric form from a monomeric form of a multimer-forming polypeptide (such as prion) that involves a treatment with trypsin. The method disclosed in WO 2006/088281 A1 differs from the currently disclosed method. WO 2006/088281 A1 specifically promoted the using of antibodies against non-repeated sequence in prion protein over repeated sequences such as the octarepeat sequence, because antibodies against the octarepeat sequence produced higher background using the method disclosed in this publication.

In a recent publication, the protease thermolysin was used for diagnosis of prion diseases (See Owen et al., (2007) Mol. Biotechnol. 35:161-170). Thermolysin specifically cleaves at several hydrophobic residues, residues that are absent from the protease accessible amino-terminal region of PrP^{Sc}.

The present invention thus provides an assay method, comprising the steps of:
(a) obtaining a sample suspected of containing a pathogenic form of prion protein (PrP^{Sc}) which has a protease-resistant core and an octarepeat region, wherein said sample may or may not contain a normal form of prion protein (PrP^{C});
(b) treating said sample with at least one site-specific protease wherein said at least one site-specific protease is trypsin or S-V8, under conditions in which proteolytic digestion of said PrP^{Sc} and said PrP^{C}, if present, is substantially complete,
   i. wherein said PrP^{Sc} has no cleavage site for said protease within said octarepeat region or between said octarepeat region and said protease-resistant core whereby a fragment of amino-proximal region including said octarepeat region remains connected to said protease-resistant core after said substantially complete proteolytic digestion,
      and
   ii. wherein said PrP^{C} has at least one available cleavage site for said protease within amino acid region corresponding to said protease-resistant core, and said at least one available cleavage site is cleaved by said substantially complete proteolytic digestion;
(c) preventing any further proteolytic digestion following said substantially complete proteolytic digestion in (b) by adding a protease inhibitor or removing said protease;
(d) denaturing said site-specific-protease-treated PrP^{Sc} thereby providing denatured PrP^{Sc}; and
(e) detecting the presence of said denatured PrP^{Sc} using at least two binding partners, a first binding partner and a second binding partner,
   i. wherein said first binding partner specifically binds a first epitope which is located within said fragment of amino-proximal region that remains connected to said protease-resistant core after said substantially complete proteolytic digestion, and
   ii. wherein said second binding partner specifically binds a second epitope which is located within said protease-resistant core, wherein said second epitope is in a region of said PrP^{C} that is separated from said first epitope after said substantially complete proteolytic digestion.

The present invention also provides a kit for detecting the presence of a pathogenic form of prion protein (PrP^{Sc}) which has a protease-resistant core and an octarepeat region in a sample suspected of containing said PrP^{Sc}, wherein said sample may or may not contain a normal form of prion protein (PrP^{C}), the kit comprising:
(a) at least one site-specific protease, wherein said at least one site-specific protease is trypsin or S-V8,
   i. wherein said PrP^{Sc} has no cleavage site for said protease within said octarepeat region or between said octarepeat region and said protease-resistant core whereby a fragment of amino-proximal region including said octarepeat region remains connected to said protease-resistant core after a substantially complete proteolytic digestion of PrP^{Sc} by said protease, and
   ii. wherein said PrP^{C} has at least one available cleavage site for said protease within amino acid region corresponding to said protease-resistant core;
(b) optionally, a protease inhibitor which is capable of inhibiting the activity of said protease;
(c) optionally, a denaturant which is capable of denaturing said PrP^{Sc};
(d) at least two binding partners, a first binding partner and a second binding partner,
   i. wherein said first binding partner specifically binds a first epitope which is located within said fragment of amino-proximal region that remains connected to said protease-resistant core after a substantially complete proteolytic digestion of said PrP^{Sc} by said protease, and
   ii. wherein said second binding partner specifically binds a second epitope which is located within said protease-resistant core, wherein said second epitope is in a region of said PrP^{C} that is separated from said first epitope after a substantially complete proteolytic digestion of said PrP^{C} by said protease; and
(e) an instruction for using said kit to detect the presence of any pathogenic form of prion protein.

### Site-Specific Protease

Site-specific proteases that are useful in the present invention are proteases that cleave peptide bonds of specific, discrete amino acid residues. The site-specific proteases will cleave a protein at one type or a small number of specific amino acid residues thus allowing predictability in the cleavage of the prion protein. Said site-specific proteases are: trypsin which is a site-specific protease that cleaves on the carboxyl side of Lys (K) or Arg (R) residues, when the amino acid on the carboxyl side of K and R is not Pro, and S-V8 which is a site-specific protease from *S. aureus* V8 strain that cleaves on the carboxyl side of Asp (D) or Glu (E) residues. For specificity of trypsin cleavage, see, e.g., Neurath et al. (1950) Chem. Rev. 46:70; Walsh and Neurath (1964) Proc.Natl. Acad Sci. USA 52:884-889; Walsh (1970) Meth. Enzymol. 19:41. For specificity of S-V8 cleavage, see, e.g., Drapeau et al. (1972) J. Biol. Chem. 247:6720-6726, and Houmard et al. (1972) Proc. Natl. Acad Sci. USA 69:3506-3509. Trypsin and S-V8 are commercially available from various suppliers such as Pierce, part of Thermo Fisher Scientific Inc., Rockford IL, and Sigma-Aldrich, Inc., St. Louis, MO.

Other such site-specific proteases can be readily selected by one of ordinary skill in the art.

In addition, to be useful in the present method, there must be a cleavage site for the site-specific protease in the prion protein in the region between the epitopes recognized by the two binding partners used in the assay method. At least one protease cleavage site will be within the protease-resistant core region (approximately amino acids 90-231 of the prion protein) and this site will be cleaved by the site-specific protease only when the prion protein is in the PrP^{C} form and not when the prion protein is in the PrP^{Sc} form. Preferably at least one of the epitopes will be in the protease-resistant core region of the prion protein. Preferably, at least one other epitope will be located within the amino-proximal region of the prion protein, and more preferably, within the octarepeat region of the prion protein. Preferably, the site-specific protease does not cleave at a site within the octarepeat region of the prion protein. The core repeated sequence of the octarepeat region is GQPHGG(G/S)(-/G)W (SEQ ID NO: 11), which can vary slightly in prions from different species (See Figures 1 A and 1 B or 2A and 2B for the sequences of 10 different prion proteins showing the octarepeat sequences.). In some literature, the core repeated sequence of the octarepeat region was disclosed as P(H/Q)GGG(-/T)WGQ (See, e.g., US Patent No. 7,097,997 B1) (SEQ ID NO: 12), which is a slight shift and variation of the octarepeat sequence disclosed herein, but both octarepeat sequences are apparently related to the same region. Figure 3 shows a schematic representation of the PrP^{C} and PrP^{Sc} forms showing the octarepeat regions, exemplary epitope sites and exemplary site-specific protease cleavage sites. The site-specific protease will cleave the PrP^{C} form in at least one site between the epitopes recognized by the two binding partners, preferably the anti-prion antibodies used in an ELISA. Thus, the PrP^{C} form will not be detected in the ELISA. The PrP^{Sc} form, however, will not be cleaved by the site-specific protease in the region between the two epitopes because the conformation of this isoform makes the sites unavailable for protease cleavage. Thus, the PrP^{Sc} will be detectable in the ELISA.

### Protease Treatment

As described above, the samples to be analyzed may naturally contain high levels of PrP^{C}. PK has been used in other settings to digest the PrP^{C} form, leaving the more resistant PrP^{Sc} form. However, PrP^{Sc} is not completely resistant to proteolysis if high concentrations of PK and/or prolonged exposure times are used as shown by the fact that PK treatment reduces infectivity of the pathogenic form. See, McKinley et al. (1983) Cell 35:57-62. Therefore, PK treatment must be carefully controlled in order to provide complete cleavage of the PrP^{C} form but leave the resistant core of the PrP^{Sc} form intact. Too little PK digestion will leave residual PrP^{C} form which will yield a false positive in the detection phase and too much PK digestion will cleave the PrP^{Sc} resistant core making it undetectable in the detection phase. In addition, although the particular PK digestion site(s) of PrP^{Sc} vary since the pathogenic form can adopt multiple conformations, PK digestion of PrP^{Sc} typically results in multiple protease resistant fragments around residues 90-231, which may reduce or eliminate the binding of anti-prion antibodies directed against epitopes in this region. See, Telling et al. (1996) Science 274:2079-2082.

In the current disclosure, a sample suspected of containing a pathogenic form of prion protein is treated with the selected site-specific protease under conditions in which any non-pathogenic prion protein would be digested substantially completely. One of ordinary skill in the art is competent to determine the appropriate conditions. Conditions of substantially complete digestion can readily be determined by tests using recombinant PrP. For trypsin as the site-specific protease, typically a trypsin concentration of 50 µg/ml for 1 hour at 37°C in TBST (50 mM Tris-HCl, pH 7.5, 150 mM NaCl, 0.05% Tween 20), preferably with 1% Triton X-100 and 0.2 M CaCl₂, and a trypsin concentration of 10 µg/ml for 1 hour at 37°C in TBSS (50 mM Tris-HCl, pH 7.5, 150 mM NaCl, 2% Sarkosyl), are both adequate.

Following substantially complete digestion of the non-pathogenic prion protein, the site-specific protease must be removed, inactivated or inhibited in order to prevent any further protease digestion, for instance, of the anti-prion antibodies that will be used for detection. The protease may be inhibited by the addition of one or more protease inhibitors. Protease inhibitors are well known in the art and include phenylmethylsulfonyl fluoride (PMSF), aprotinin, diisopropylfluorophosphate (DFP), and 1-chloro-3-tosylamido-4-phenyl-2-butanone (TLCK), among others. Alternately, some proteases are available in an immobilized form (e.g., in an agarose matrix) which can be readily removed from the reaction by conventional means (e.g., centrifugation, filtration, etc.).

### Denaturation

Following the digestion of the sample by the site-specific protease and inhibition/removal of the protease, the digested sample which is suspected of containing PrP^{Sc} is denatured in order for a sensitive detection of epitopes.

The denaturation can be accomplished in a number of ways. In one embodiment, a chaotropic agent, preferably a guanidinium compound, e.g., guanidine thiocyanate or guanidine hydrochloride, is added to a concentration of between 3M and 6M. Addition of the chaotropic agent in these concentrations causes the pathogenic prion protein to denature. For this embodiment, the chaotropic agent must be removed or diluted before the detection is carried out because it will interfere with the binding of the binding partners to prion epitopes, e.g., anti-prion antibodies used in the ELISA.

In another embodiment, the denaturation is accomplished by either raising the pH to 12 or above ("high BH") or lowering the pH to 2 or below ("low pH"). Details of the pH dissociation/denaturation technique are described in PCT/US2006/001437 and US application number 11/518,091.

Exposure of un-denatured PrP^{Sc} to either high or low pH results in the denaturation of the pathogenic prion protein. In this embodiment, exposure of PrP^{Sc} to high pH is preferred. A pH between 12.0 and 13.0 is generally sufficient; preferably, a pH of between 12.5 and 13.0 is used; more preferably, a pH of 12.7 to 12.9; most preferably a pH of 12.9. Alternatively, exposure of PrP^{Sc} to a low pH can be used to denature the pathogenic prion protein. For this alternative, a pH of between 1.0 and 2.0 is sufficient. Exposure of the protease-digested sample to either a high pH or a low pH is carried out for only a short time e.g. 60 minutes, preferably for no more than 15 minutes, more preferably for no more than 10 minutes. Longer exposures than this can result in significant deterioration of the structure of the pathogenic prion protein such that epitopes recognized by binding partners used in the detection steps are destroyed.

After exposure for sufficient time to denature PrP^{Sc}, the pH can be readily readjusted to neutral (that is, pH of between about 7.0 and 7.5) by addition of either an acidic reagent (if high pH denaturation conditions are used) or a basic reagent (if low pH denaturation conditions are used). One of ordinary skill in the art can readily determine appropriate protocols and examples are described herein. Because the high pH or low pH used in the denaturation step can be easily readjusted to neutral by addition of small volumes of suitable acid or base, this embodiment allows the use directly in the detection step, e.g., ELISA, without any additional washes and without increasing the sample volumes significantly.

In general, to effect a high pH denaturation condition, addition of NaOH to a concentration of about 0.05 N to about 0.2 N is sufficient. Preferably, NaOH is added to a concentration of between 0.05 N to 0.15 N; more preferably, 0.1 N NaOH is used. Once the denaturation of PrP^{Sc} is accomplished, the pH can be readjusted to neutral (that is, between about 7.0 and 7.5) by addition of suitable amounts of an acidic solution, e.g., phosphoric acid, sodium phosphate monobasic.

In general, to effect a low pH denaturation condition, addition of H₃PO₄ to a concentration of about 0.2 M to about 0.7 M is sufficient. Preferably, H₃PO₄ is added to a concentration of between 0.3 M and 0.6 M; more preferably, 0.5 M H₃PO₄ is used. Once the denaturation of PrP^{Sc} is accomplished, the pH can be readjusted to neutral (that is, between about 7.0 and 7.5) by addition of suitable amounts of a basic solution, e.g., NaOH or KOH.

The denatured samples can be used for further detection of PrP^{Sc}

### Detection

Denatured PrP^{Sc} can be detected with at least two binding partners specific for prion sequences, with one binding partner specifically binding an epitope in the amino-proximal region of denatured PrP^{Sc}, preferably in the octarepeat region, and the other binding partner specifically binding an epitope in the protease-resistant core region of denatured PrP^{Sc} which is no longer available in PrP^{C} due to protease cleavage.

In certain embodiments of the method, the binding partners are antibodies, and a preferred embodiment among these has its detection step carried out using an ELISA.

In certain other embodiments, the binding partners used in the method are aptamers specific to the first epitope and the second epitope.

Antibodies, modified antibodies and other reagents, that bind to prions, particularly to PrP^{C} or to the denatured PrP, have been described and some of these are available commercially (see, e.g., anti-prion antibodies described in Peretz et al. (1997) J. Mol. Biol. 273:614; Peretz et al. (2001) Nature 412:739; Williamson et al. (1998) J. Virol. 72:9413; Polymenidou et al. (2005) *supra*; U.S. Patent No. 6,765,088). Some of these and others are available commercially from, inter alia, In Pro Biotechnology, South San Francisco, CA, Cayman Chemicals, Ann Arbor MI; Prionics AG, Zurich; also see, WO 03/085086 for description of modified antibodies.

Preferred anti-prion antibodies will be ones that bind to a denatured form of the pathogenic prion.

Particularly preferred first antibodies will be ones for the first epitope that is located within the octarepeat region of the prion protein. Examples of such antibodies are POM2, POM11, POM12, POM14, 3B5, 4F2, 13F10, SAF-15, SAF-31, SAF-32, SAF-33, SAF-34, SAF-35, and SAF-37. (See, e.g., Polymenidou et al. (2005) Lancet Neurol. 4:805-814; Krasemann et al. (1996) Mol. Medicine 2:725-734; Féraudet, et al. (2005) J. Biol. Chem. 280:11247-11258; U.S. Patent No. 7,097,997 B1.)

In certain embodiments, the first antibody will bind to an epitope that is outside of the octarepeat region. Exemplary first binding partners in these embodiments include monoclonal antibodies such as BAR210, BAR231, and 14D3 (See, e.g., Krasemann et al. (1996) Mol. Medicine 2:725-734; Féraudet, et al. (2005) J. Biol. Chem. 280:11247-11258).

Preferred second antibodies will be ones that recognize the second epitopes that are within the protease-resistant core region of PrP^{Sc} and that are no longer available in PrP^{C} due to protease cleavage. Because the first cleavage site in the protease-resistant core region of PrP^{C} for trypsin is different from that for S-V8 (K-106 for trypsin and D-144 for S-V8, numbered according to human PrP), there will be more useful epitopes for trypsin-digested PrP^{Sc} than for S-V8.

Exemplary second binding partners in embodiments that are digested with trypsin include monoclonal antibodies such as 3F4 (US Patent No. 4,806,627), POM1, POM4, POM5, POM6, POM7, POM8, POM9, POM10, POM13, POM15, POM16, POM17, POM19 (for POM antibodies, see, Polymenidou et al. (2005) Lancet Neurol. 4:805-814), SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10 (Krasemann et al. (1996) Mol. Medicine 2:725-734; Féraudet, et al. (2005) J. Biol. Chem. 280:11247-11258; U.S. Patent No. 7,097,997 B1.), D18 (Peretz et al. (1997) J.Mo/ Biol. 273:614), 6H4 (Liu et al. (2003) J. Histochem. Cytochem. 51:1065), and BDI115 (Biodesign International). The 3F4 antibody recognizes an epitope, MKHM, at amino acids 109-112 of human PrP. Other antibodies recognize various epitopes within the regions from about amino acid 107 to about amino acid 231 numbered according to human PrP species.

In certain embodiments of the method in which trypsin is used, the second binding partners specifically bind to epitopes located within the globular domain of PrP (i.e., from about amino acid 122 to about amino acid 231 of PrP). Exemplary second binding partners in these embodiments include monoclonal antibodies such as POM1, POM4, POM5, POM6, POM7, POM8, POM9, POM 10, POM13, POM15, POM16, POM 17, POM 19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, and BDI115.

Exemplary second binding partners in embodiments that are digested with S-V8 include monoclonal antibodies such as SAF-53, SAF-54, SAF-60, SAF-61, SAF-66, SAF-69, SAF-70, SAF-75, SAF-76, Pri917, BAR234, Sha31, 11B9, 12F10, 6H4, and POM5.

Other anti-prion antibodies can readily be generated by methods that are well-known in the art.

One of skill in the art will appreciate from the disclosure herein that the first and second antibodies are selected such that the first antibody specifically binds a first epitope in the amino-proximal region, preferably in the octarepeat region, and the second antibody specifically binds a second epitope within a region of the protease-resistant core of PrP^{Sc} that has been cleaved away in PrP^{C}. In this way, following digestion with the site-specific protease, the epitopes recognized by the first and second antibodies will be present on different fragments of the PrP^{C} (and so will not be capable of detection in the Sandwich ELISA) but these epitopes will be present on a single fragment of the PrP^{Sc} (and so will be detectable in the Sandwich ELISA).

Some anti-prion antibodies are specific for prion protein from one or a limited number of animal species, others are capable of binding prion proteins from many animal species. It will be apparent to choose suitable anti-prion antibodies based upon the samples to be analyzed and the purpose of the testing.

The protease-digested and denatured pathogenic prion proteins are preferably detected in an ELISA type assay, either as a direct ELISA or an antibody Sandwich ELISA type assay, which are described more fully below. Although the term "ELISA" is used to describe the detection with anti-prion antibodies, the assay is not limited to ones in which the antibodies are "enzyme-linked." The detection antibodies can be labeled with any of the detectable labels described herein and well-known in the immunoassay art.

In a preferred embodiment of the method, the protease-digested and denatured pathogenic prion proteins are detected using an antibody Sandwich type ELISA. In this embodiment, the denatured prion protein is captured on a solid support comprising a capture antibody, which can be the first antibody or the second antibody in different embodiments. The solid support with the captured prion protein, is optionally washed to remove any unbound materials, and then contacted with a detection antibody, which can be the second antibody or the first antibody, depending on what the capture antibody is (the capture antibody is different from the detection antibody), under conditions that allow the detection antibody to bind to the captured prion protein. Methods for carrying out said Sandwich ELISAs are well known and are described with the Examples herein.

Suitable solid supports include any material that is an insoluble matrix and has a rigid or semi-rigid surface to which the pathogenic-prion specific reagent can be linked or attached. Exemplary solid supports include, but are not limited to, substrates such as nitrocellulose, polyvinylchloride, polypropylene, polystyrene, latex, polycarbonate, nylon, dextran, chitin, sand, silica, pumice, agarose, cellulose, glass, metal, polyacrylamide, silicon, rubber, polysaccharides, polyvinyl fluoride; diazotized paper; activated beads, magnetically responsive beads, and any materials commonly used for solid phase synthesis, affinity separations, purifications, hybridization reactions, immunoassays and other such applications. The support can be particulate or can be in the form of a continuous surface and includes membranes, mesh, plates, pellets, slides, disks, capillaries, hollow fibers, needles, pins, chips, solid fibers, gels (e.g. silica gels) and beads, (e.g., pore-glass beads, silica gels, polystyrene beads optionally cross-linked with divinylbenzene, grafted co-poly beads, polyacrylamide beads, latex beads, dimethylacrylamide beads optionally crosslinked with N-N'-bis-acryloylethylenediamine, iron oxide magnetic beads, and glass particles coated with a hydrophobic polymer. Preferred solid support for the first antibody is a microtiter plate.

Preferably, either the capture antibody or the detection antibody in the Sandwich ELISA recognizes an epitope within the octarepeat region of the prion protein. In some embodiments, the detection antibody is detectably labeled; in further embodiments, the detection antibody is enzyme labeled.

In certain other embodiments, the binding partners used in the method are aptamers. Aptamers are nucleic acids having the molecular recognition properties of antibodies. Aptamers are smaller and less complex than antibodies, and thus maybe easier to manufacture and modify. (See, e.g., Osbome et al. (1997) Curr. Opin. Chem. Biol. 1:5-9; Bunka et al. (2006) Nat. Rev. Microbiol. 4:588-596.) Methods of generating aptamers specific to protein epitopes are readily available (see, e.g., Tuerk and Gold (1990) Science 249:505-510; Cox et al. (1998) Biotechnol Prog. 14:845-850; Cox et al. (2002) Nucleic Acids Res. 30:e108). Aptamers can be linked to enzymes that have an easily detectable activity (see, e.g., Drolet et al. (1996) Nat. Biotechnol. 14:1021).

Any of the detection methods for a pathogenic prion described hereinabove can be used in a method to diagnose a prion-related disease in any sample.

For use in the methods described herein, the sample can be anything known to, or suspected of, containing a pathogenic prion protein. In some embodiments, the sample is a biological sample (that is, a sample prepared from a living or once-living organism), or a non-biological sample. In some embodiments, the sample is a biological sample. Non-limiting examples of biological samples are organs (e.g., brain, liver, and kidney), cells, whole blood, blood fractions, blood components, plasma, platelets, serum, cerebrospinal fluid (CSF), brain tissue, nervous system tissue, muscle tissue, muscle and fatty tissue (e.g., flesh), bone marrow, urine, tears, non-nervous system tissue, biopsies, necropsies, foods that are sourced from a living or once-living organism, and any other organic matter such as plant materials. In some embodiments, the biological sample comprises whole blood, blood products, blood fractions, blood components, plasma, platelets, red blood cells or serum. The biological sample can be obtained during a health related procedure such as a blood donation or screening, biopsy, autopsy, or necropsy, or during a process or procedure during food preparation such as animal selection and slaughter and quality assurance testing of finish product. In some embodiments, the sample is non-biological. Non-limiting examples of non-biological samples include pharmaceuticals, cosmetics and personal care products, contaminated instruments and foods that are not sourced from a living or once-living organism, and the like.

Suitable controls can also be used in the assays described herein. For instance, a negative control of PrP^{C} can be used in the assays. A positive control of PrP^{Sc} (or PrPres) could also be used in the assays. Such controls can optionally be detectably labeled.

### Kits

The above-described assay reagents, including the site-specific proteases, protease inhibitors (optional), denaturing agents (or denaturants, optional), anti-prion binding partners such as antibodies, etc., can be provided in kits, with suitable instructions and other necessary reagents, in order to conduct detection assays as described above. The kit may further contain suitable positive and negative controls, as described above. The kit can also contain, depending on the particular detection assay used, suitable labels and other packaged reagents and materials (i.e., wash buffers and the like).

### EXAMPLES

In order that the invention disclosed herein can be more efficiently understood, examples are provided below. It should be understood that these examples are for illustrative purposes only and are not to be construed as limiting the invention in any manner.

### Example 1

### Coating 3F4 Lyophilized Plates

The commercially available monoclonal antibody 3F4 (Covance Research Products, Inc.) was used as a capture antibody in several examples shown below. It specifically recognizes residues 109-112 (amino acids MKHM) which is located in the protease-resistant core region of human prion protein. It was diluted at 2.5 µg/mL in coating buffer (0.1 M NaHCO₃, pH 8.9) and incubated at room temperature (RT) for 5min with agitation. The diluted antibody 3F4 was added to Thermo Labsystem MicroLite 2+ microplates at volumes of 150 µL/well, and then incubated at RT for over night in a plastic wrap sealed tray with moisture. The plates were washed 3 times with Tris-buffered Saline with Tween 20 (TBST, 50 mM Tris-HCl, pH 7.5, 150 mM NaCl, 0.05% Tween 20) on a BioTek Elx450 washer. A blocking buffer (0.01 x BlockerCasein in Tris-buffered Saline (TBS, 50 mM Tris-HCl, pH 7.5, 150 mM NaCl) with 3% sucrose) was then dispensed to the plates at volumes of 300 µL/well of on the BioTek Elx450 washer, and incubated at RT for 1hr. The plates were lyophilized for overnight at 16°C, and each plate was put into a pouch with 3 desiccants, double-sealed and stored at 4°C. In general, all the coated plates were blocked with 0.02% casein for 1hr at 37°C prior to use.

### Example 2

### Trypsin Digestion of Normal Human Plasma Samples

### (A) Digestion of plasma with trypsin

To test whether trypsin degrades PrP^{C} and renders it undetectable by ELISA, 10% normal human plasma in TBST was treated with increasing concentrations of trypsin (Table 1). The trypsin used in this and following examples was obtained from Pierce (TPCK Trypsin, Catalog No. 20233). The digestion was incubated at 37°C for 1 hr with shaking at 200 rpm. Digestion was stopped by addition of the protease inhibitor phenylmethylsulphonyl fluoride (PMSF, final concentration 1 mM) with incubation at RT for 15 min. The trypsin-treated samples were diluted to 1% (of the normal human plasma), and 150 µL was used in each well for ELISA.

### (B) ELISA analysis for PrP^{C}

Samples were tested for the presence of PrP^{C} by a Sandwich ELISA using 3F4 as capture antibody and detection with POM2 antibody (Polymenidou et al., supra) conjugated to alkaline phosphatase (AP) using a chemiluminescence substrate for light detection. POM2 specifically recognizes the octarepeat epitopes within residues 59-91.

Briefly, trypsin-treated plasma samples were diluted to 1% (of the original human plasma) in TBST and transferred to 3F4-coated plates at volumes of 150 µL/well. The plates were incubated at 37°C for 1 hr with shaking at 400 rpm, and then washed 6 times with TBST. AP-conjugated POM2 detection antibody (0.01 µg/mL) was added into the plates at volumes of 150 µL/well and incubated at 37°C for 1 hr. The plates were again washed 6 times with TBST. Finally, 150 µL/well of Lumi-Phos Plus substrate with 0.05% SDS was added to the plates and incubated at 37°C for 30 min, and signals were read with a microplate luminometer. The results are shown in Table 1, and measurement units are defined in relative light units (RLU).

**TABLE 1**

| **Trypsin (µg/mL)** | **Relative Amount of PrP^{C} Detected in Human Plasma (RLU)** | | |
|---|---|---|---|
| | **Ave** | **SD** | **CV (%)** |
| **0** | **195.4** | 3.1 | 1.6 |
| **100** | **70.5** | 1.6 | 2.3 |
| **200** | **10.0** | 0.3 | 2.6 |
| **300** | **8.5** | 0.5 | 6.2 |
| **400** | **1.9** | 0.1 | 5.0 |
| **500** | **1.2** | 0.4 | 29.8 |

| | | | |
|---|---|---|---|
| Ave: Average. SD: standard deviation. CV: coefficient of variation. | | | |

The result shows that trypsin digested PrP^{C} in human plasma and that detection of PrP^{C} was abolished in samples treated with higher concentration of trypsin (Table 1). We found dose response between levels of detected PrP^{C} and trypsin concentration. At trypsin concentration of 400 µg/mL, levels of PrP^{C} dropped 100 fold to background levels.

### Example 3

### Trypsin Digestion of Normal Syrian Hamster Brain Homogenate Samples

In this example, we tested trypsin digestion of PrP^{C} present in normal Syrian hamster (SHa) brain homogenate (BH).

Five micro-liters of 10% SHa BH (w/v) was mixed with 5 µL of trypsin in TBST with 1% Triton X-100 and 0.2 M CaCl₂, in increasing concentrations of trypsin (Table 2), for 1 hr at 37°C with shaking at 750 rpm. Each digested sample was diluted by addition of 140 µL of TBS, and digestion was stopped by addition of 1.5 µl/well of 100 mM PMSF (for final PMSF concentration 1 mM) and incubation at RT for 10 min with shaking at 750 rpm.

The trypsin-digested SHa BH samples were tested for the presence of PrP^{C} by a Sandwich ELISA using 3F4 as capture antibody and detection with AP-conjugated POM2 antibody as described in Example 2. The digestion solutions, 150 µL of each, were transferred into each well of the 3F4-coated microplates and incubated at 37°C for 1 hr with shaking at 300 rpm. Plates were washed with TBST, and 150 µL/well of 0.01 µg/mL of AP-conjugated POM2 in 0.01 x casein-TBST was added and incubated at 37°C for 1 hr. Substrate was added and plates were read with a microplate luminometer as described in Example 2. The results are shown in Table 2.

**TABLE 2**

| **Trypsin µg/mL** | **Relative Amount of PrP^{C} Detected in Syrian Hamster Brain Homogenate (RLU)** | | | |
|---|---|---|---|---|
| | **Average** | **SD** | **CV (%)** | **Removal (%)** |
| **100** | **11.7** | 2.0 | 17.5 | 99.60 |
| **50** | **14.9** | 0.7 | 4.6 | 99.49 |
| **25** | **20.5** | 1.7 | 8.5 | 99.30 |
| **0** | **2937.9** | 71.1 | 2.4 | 0.00 |

Similar to the results with human plasma, trypsin digested PrP^{C} in SHa brain homogenate and detection of PrP^{C} was also abolished (Table 2). In contrast to plasma, efficient degradation of PrP^{C} in brain homogenate was achieved with trypsin concentration of 25 µg/mL, much lower than the trypsin concentration needed for efficient degradation of PrP^{C} in plasma.

### Example 4

### Trypsin and PK Digestion of Syrian Hamster Brain Homogenate Samples and Detection with POM and 3F4 Antibodies

In this example, we compared the effects of trypsin and PK digestion on ELISA detection of Syrian Hamster PrP^{Sc}, a pathogenic form of prion, using 4 different pairs of monoclonal antibodies and one concentration of PK and trypsin.

### (A) Digestion of SHa BH with trypsin or PK and denaturation of digested samples

Two micro-liters of 5% SHa BH samples (normal or infectious) were treated with trypsin or PK at the same concentration of 25 µg/mL, each in final reaction volume of 2 µL, for 1 hr at 37°C. Digestion was stopped with PMSF (2 mM, at RT for 10 min).

Samples were denatured with guanidine hydrochloride (GdnHCl) to ensure maximum binding of antibodies to PrP^{Sc} (3 M final concentration, 1 hr at RT). Denatured samples were then diluted to 0.1 M GdnHCl and added to ELISA plates coated with various mAbs.

### (B) ELISA analysis for PrP^{Sc}

Microplates were coated with mAb 3F4, POM2 or POM17 at 150 µL of 2.5 µg/mL in 0.1 M NaHCO₃, pH 8.9, at 4°C overnight. The plates were blocked with 0.02% casein at 37°C for 1 hr prior to use. POM17 recognizes an epitope within the protease resistant fragment of residues ~90-231, in particular an epitope within the globular domain of residues ~122-231.

The protease-digested and subsequently denatured SHa BH samples were transferred to plates coated with 3F4, POM2 or POM17 at the final volumes of 150 µL/well. The plates were incubated at 37°C for 1hr with shaking at 300 rpm, and then washed with TBST. AP-conjugated POM2 (POM2AP) or AP-conjugated POM 17 (POM17AP), both at final concentration of 0.01 µg/mL, was added into the plates at the final volumes of 150 µL/well and incubated at 37°C for 1 hr. The plates were again washed with TBST, Lumi-Phos Plus substrate was added and incubated at 37°C for 30 min, and signals were read as shown in Example 2. The results of this Sandwich ELISA experiment are shown in Table 3.

**TABLE 3**

| Antibody Combination | Antibody | | | Relative Amount of PrP Detected in Normal Brain Homogenate (RLU) | | | | Relative Amount of PrP Detected in Infectious Brain Homogenate (RLU) | | | |
|---|---|---|---|---|---|---|---|---|---|---|---|
| | **Epitope for Capture** | **Capture Antibody** | **Detection Antibody** | **Trypsin** | | **PK** | | **Trypsin** | | **PK** | |
| | | | | Ave | SD | Ave | SD | Ave | SD | Ave | SD |
| **1** | 109-112 | **3F4** | **POM2AP** | 21.4 | 1 | 10 | 0.1 | 2370.3 | 37.6 | 527 | 5.9 |
| **2** | 109-112 | **3F4** | **POM17AP** | 2 | 0.3 | 1.7 | 0.4 | 253.7 | 4.4 | 200.2 | 5.7 |
| **3** | 59-91 | **POM2** | **POM17AP** | 14.6 | 1.8 | 1.8 | 0.5 | 381 | 7.6 | 68.4 | 5.5 |
| **4** | 121-231 | **POM17** | **POM2AP** | 70.6 | 8.5 | 13.4 | 0.4 | 2248.3 | 24 | 596.2 | 40.3 |

We found from the results shown in Table 3 that PK reduced detection signal in all cases as compared to trypsin-digested samples. Reduction of more than 70% was observed between trypsin and PK-digested samples when we used POM2 either as detection antibody or capture antibody in the infectious brain homogenates (rows 1, 3 and 4). For example the signal obtained with 3F4/POM2AP (capture/detection) was reduced from 2370 to 527 RLU. A similar proportion in signal reduction was observed with POM2/POM17AP and POM17/POM2AP. The reduction in signal is due to the PK digestion of the octarepeat epitopes recognized by POM2 within residues 23-89.

We also noticed a drop of 20% in signal when we treated the infectious brain homogenate samples with PK and detected with 3F4/POM 17AP, even though the epitopes for these antibodies are in the protease-resistant core. This drop in signal suggests that even limited PK digestion can affect the protease resistant fragment 90-231. It is worth noting that the highest readings were observed when POM2, which specifically binds to the octarepeat region of PrP, was used as the detection antibody after trypsin digestion. The next highest reading was observed when POM2 was used as the capture antibody. We conclude that, in the presence of 4 octarepeats, POM2 binds more than one epitope and therefore has stronger binding than antibodies that recognize only one epitope, such as POM17 and 3F4.

### Example 5

### Anti-PrP Antibody Screening Against PrP of Various Species

A number of anti-PrP antibodies as shown in Table 4, which includes SAF-32, a monoclonal antibody against the octarepeat region, were screened for their abilities to bind to recombinant PrP (rPrP) of different animal species and to mouse PrP^{Sc}.

**TABLE 4: Information of Antibodies Used in the Screening Test**

| **Antibody Name** | **Manufacturer** | **Cat #** | **Host** | **Immunogen (Specificity)** |
|---|---|---|---|---|
| HuM D18 | InPro | ABR-0D18 | humanized | AMSRPMMHFGNDWEDRYYR ENMNRY (amino acids 133-157) |
| HuM D13 | InPro | ABR-0D13 | humanized | HNQWNKPSKPK (amino acids 96-106) |
| mAb 6H4 | Prionics | 01-010 | mouse | DYEDRYYRE (amino acids 144-152) |
| Mouse anti Prion (MAB5424) | Chemicon | MAB5424 | mouse | Recombinant PrP amino acids 23-237 |
| mAb to prion 7D9 | Biodesign | Q06850M | mouse | Recombinant mouse PrP (amino acids 23-237) |
| mAb to prion BDI115 | Biodesign | Q65115M | mouse | PrP peptide (a.a. 146-159 of bovine prion protein) coupled to a carrier protein |
| **Mouse anti Prion (SAF-32)** | **Cayman Chemical** | **189720** | **mouse** | **SAF of inf hamster (specific to the octarepeat region)** |
| Mouse anti Prion (SAF-53) | Cayman Chemical | 189730 | mouse | SAF of inf hamster (specific to amino acids 142-160) |
| Mouse anti Prion (SAF-61) | Cayman Chemical | 189755 | mouse | SAF of inf hamster (specific to amino acids 142-160) |
| Mouse anti Prion (SAF-83) | Cayman Chemical | 189765 | mouse | SAF of inf hamster (recognized amino acids 126-164 but not 142-160 ^{a}) |

| | | | | |
|---|---|---|---|---|
| ^{a} Information from Feraudet, et al. (2005) J. Biol. Chem. 280:11247-11258 and U.S. Patent No. 7,097,997 B1. | | | | |

### (A) Coating plates with various PrP

Some microplates were coated with recombinant PrPs (rPrPs) of Bovine, Deer, Human, Sheep or Mouse following these steps. Each rPrP was diluted and denatured in 3M guanidine thiocyanate (GdnSCN) to 1, 0.1, 0.01, and 0.001 µg/ml, incubated at RT for 10 min, and 100 µl/well of the denatured rPrP was added in duplicate to 96-well plates. Then, to each well, 100 µl of 0.1 M NaHCO₃, pH 8.8, was added, and plates were incubated at 4°C for overnight.

Other microplates were coated with Mouse BH PrP^{Sc} following these steps. PrP^{Sc}-infected Mouse BH (400 µL of 10% BH) was thawed in TBS with 1% Tween 20 and 1% Triton X-100, and precipitated by centrifugation at 20,000 rpm for 1hr at 4°C. The precipitates were each washed with 1mL TBS of pH 7.5, and re-precipitated by centrifugation at 20,000 rpm for 10 min at 4°C. The pellets were each re-suspended in 4 mL of 3 M GdnSCN to reach the final concentration of 1% BH, and were denatured at RT for 15 min. The denatured BH samples were diluted to 0.1%, 0.01%, and 0.001% in 3M GdnSCN, and 100 µl/well of each concentration of BH was added to the plates in duplicate. Then 100 µl/well of 0.1 M NaHCO₃, pH 8.8, was added to each well of BH samples. Incubation was carried out at 4°C for overnight.

### (B) ELISA assay to detect binding of various antibodies to the coated PrPs

Each plate was washed 3 times with TBST. Each well was then blocked with 200 µL of 3% BSA in TBS and incubated at 37°C for 1 hr. After the buffer was aspirated, 100 µL/well of primary antibody to be screened was added at 0.5 µg/ml in TBS with 1% BSA, and plates were incubated at 37°C for 2 hr. After plates were washed 6 times with TBST, 100 µL/well of AP-conjugated secondary antibodies which bind to the screened primary antibodies, 1:5000 diluted in TBS with 1% BSA, were added. Plates were incubated at 37°C for 1 hr, and again washed 6 times with TBST. One hundred µL/well of Lumi-Phos Plus substrate with 0.05% SDS was added and incubated at 37°C for 30 min. Signal was read with a luminometer, and the results of two of the different coated amounts of each PrP, 0.1 ng and 1 ng per assay for each rPrP, as well as 0.1 mg and 1 mg per assay for Mouse BH PrP^{Sc}, are shown in Table 5.

The results in Table 5 show that SAF-32, which specifically recognizes the octarepeat region, bound PrP proteins of all the animal species tested - bovine, deer, human, sheep, and mouse. The strongest bindings of SAF-32 were to rPrP of bovine, deer, human and sheep. The results also showed significant binding between SAF-32 and mouse PrP^{Sc} in the brain homogenates.

**TABLE 5: Relative Amount of PrP Bound by Various Antibodies (RLU)**

| **Antibody** | **Bovine rPrP** | | **Deer rPrP** | | **Human rPrP** | | **Sheep rPrP** | | **Mouse rPrP** | | **Mouse BH PrP^{Sc}** | |
|---|---|---|---|---|---|---|---|---|---|---|---|---|
| | 0.1 ng /assay | 1 ng /assay | 0.1 ng /assay | 1 ng /assay | 0.1 ng /assay | 1 ng /assay | 0.1 ng /assay | 1 ng /assay | 0.1 ng /assay | 1 ng /assay | 0.1 mg BH /assay | 1 mg BH /assay |
| | 1 ng/ml | 10 ng/ml | 1 ng/ml | 10 ng/ml | 1 ng/ml | 10 ng/ml | 1 ng/ml | 10 ng/ml | 1 ng/ml | 10 ng/ml | 0.001% w/v | 0.01% w/v |
| **D18** | 109.4 | 132.5 | 134.0 | 121.0 | 77.5 | 94.7 | 168.6 | 185.6 | 366.1 | 1766.6 | 375.6 | 2230.8 |
| **D13** | 75.1 | 93.1 | 231.7 | 129.8 | 89.5 | 69.9 | 179.4 | 161.2 | 117.0 | 293.9 | 238.8 | 664.0 |
| **6H4** | 89.1 | 690.6 | 92.4 | 221.6 | 72.7 | 192.5 | 125.0 | 160.5 | 104.4 | 332.0 | 177.9 | 609.9 |
| **MAB5424** | 84.6 | 827.1 | 104.5 | 117.9 | 76.1 | 67.6 | 213.8 | 899.4 | 90.1 | 604.9 | 167.0 | 601.4 |
| **7D9** | 148.6 | 961.1 | 120.6 | 117.0 | 81.4 | 66.1 | 230.0 | 992.6 | 118.0 | 769.2 | 158.7 | 614.1 |
| **BDI115** | 59.1 | 176.9 | 104.6 | 159.5 | 48.0 | 74.3 | 108.0 | 103.7 | 54.7 | 86.7 | 118.8 | 115.6 |
| **SAF32** | **449.5** | **2397.4** | **457.1** | **2315.0** | **285.3** | **1572.3** | **293.9** | **1435.8** | **97.6** | **73.3** | **175.8** | **551.0** |
| **SAF53** | 76.6 | 145.2 | 143.8 | 263.0 | 85.2 | 93.5 | 100.1 | 153.9 | 70.8 | 611.9 | 165.2 | 667.2 |
| **SAF61** | 67.6 | 136.4 | 150.5 | 190.4 | 90.4 | 101.3 | 114.7 | 112.8 | 141.7 | 463.6 | 169.0 | 578.8 |
| **SAF83** | 45.7 | 50.8 | 121.8 | 124.8 | 65.2 | 67.0 | 98.1 | 115.4 | 181.4 | 2234.0 | 273.8 | 1725.9 |

### Example 6

### Detection of PrP in Protease-treated Human Normal or vCJD BH Samples with or without Denaturation Using SAF-32 and 3F4AP Antibodies

In this example human normal or vCJD-infected BH samples were treated with trypsin or PK, and then were either denatured or left un-denatured before being detected with SAF-32 as the capture antibody and 3F4AP as the detection antibody.

Microplates were coated with 150 µL of 3.3 µg/mL of SAF-32 diluted in 0.1 M NaH2CO3₃, pH 8.9. Plates were coated for overnight at 4°C, washed with TBST, and blocked with 1% casein for 1 hour at 37°C prior to use.

One micro-liter of 10% human normal or vCJD BH sample (w/v) was added to 1 µL of TBSTT (TBS, 1% Tween 20 and 1% Triton X-00) that has trypsin or PK for a final concentration of 25pg/mL of trypsin or PK. Digestions by the proteases were carried out for 10 min at 37°C and then stopped by 2 mM of PMSF. The digested samples were either left un-denatured or denatured with 4 M of GdnHCl at RT for 1 hour. The un-denatured samples were diluted with TBST to 150 µL. The denatured solutions were diluted with TBST to 150 uL for a final concentration of 0.1 M GdnHCl. Samples were added to the microplates coated with SAF-32 (Cayman Chemicals) at volumes of 150 µL/well for overnight at 4°C. Plates were washed with TBST, and 150 µL of 0.1 µg/mL of AP- conjugated 3F4 (Covance Research Products/Signet Labs) was added to each well. After incubation of 1 hr at 37°C, plates were washed, Lumi-Phos Plus substrate was added and incubated for 30 min at 37°C, and levels of signals were measured with a luminometer as shown in previous examples. Measurement units are defined in relative light units (RLU). The results are shown in Table 6 and Figure 4, and the ratio of results between the vCJD samples and normal samples are calculated and shown in Table 7.

**TABLE 6: Detection of PrP Using SAF-32 and 3F4AP Antibodies**

| **Condition Prior to Detection** | **Relative PrP Amount Detected in Normal Human BH (RLU)** | | | | **Relative PrP Amount Detected in vCJD-infected Human BH (RLU)** | | | |
|---|---|---|---|---|---|---|---|---|
| | **Trypsin** | | **PK** | | **Trypsin** | | **PK** | |
| | **Ave** | SD | **Ave** | SD | **Ave** | SD | **Ave** | SD |
| **Control (Un-denatured)** | **6.1** | 0.2 | **7.7** | 0.8 | **15.1** | 1.6 | **10.0** | 2.4 |
| **4M GdnHCl (Denatured)** | **50.4** | 1.5 | **18.1** | 0.4 | **1653.8** | 27.7 | **226.2** | 9.1 |

**TABLE 7: Ratio of PrP Amounts Detected in vCJD vs. Normal Samples**

| Ratio of vCJD/Normal | | |
|---|---|---|
| | Trypsin | PK |
| Control (Un-denatured) | 2.5 | 1.3 |
| 4M GdnHCl (Denatured) | 32.8 | 12.5 |

As shown in the results, treatment with trypsin or PK digested most or all PrP^{C} in normal tissue and detection was only 50.4 RLU or lower after denaturation. Un-denatured vCJD tissue did not give any significant readings. Therefore, it can be concluded that efficient detection of PrP^{Sc} by SAF-32 and 3F4 antibody combination requires denaturation of PrP^{Sc}_{.}

In addition, the results in this example showed that treatment of the vCJD BH sample with trypsin followed by denaturation resulted in a strong signal of 1653 RLU which is 32-fold higher than the signal detected in the normal BH sample. Similar treatment of the vCJD sample with PK resulted in a much lower signal of 226 RLU as compared to trypsin treatment, and the signal is 12.5-fold higher than the signal detected in the PK-treated normal sample. This example again shows that detection of PrP^{Sc} after PK digestion which cleaves within the octarepeat region in the N-terminus PrP^{Sc} is inferior to detection of PrP^{Sc} after trypsin treatment which does not affect the octarepeat region. This effect is now seen in this example and Example 4 using two different antibodies, POM2 and SAF-32, both binding the octarepeat region. This finding suggests that usage of various antibodies against the octarepeat sequence in combination with trypsin treatment will result in higher sensitivity of PrP^{Sc} detection than usage of antibodies against singular epitopes and/or PK treatment.

### Example 7

### Comparison of Digestion of Syrian Hamster Brain Homogenates with Trypsin and PK Under Various Digestion Conditions

A recent report suggests (see U. S. Patent No. 7,097,997 B1) that gentle treatment with PK could efficiently eliminate PrP^{C} while preserving all or some of the octarepeat epitopes of PrP^{Sc}, providing enhanced detection through the use of antibody that binds octarepeats. The objective of the two experiments in this example is to compare the effectiveness of PK digestion in eliminating PrP^{C} from Syrian hamster (SHa) brain homogenates (BH), while maintaining sensitivity of SHa PrP^{Sc} detection, with the method of the present invention using trypsin digestion under various digestion conditions.

Digestion of 1 µL of 10% SHa normal or scrapie-infected BH (w/v) was done with increasing amount of trypsin or PK (0 to 100 µg/mL) at 37°C for 10 min in 50 µL of TBS with 1 %Tween 20, 1 % Triton X-100 and 0.02M CaCl₂. Each digestion was stopped with PMSF at 2mM, RT for 15 min, denatured with 75 pL of 0.1 N NaOH for 10 min, and then neutralized with 30 µL of 0.3M NaH₂PO₄ for 5min at RT.

Microplates were coated with 150 µL of 2.5 µg/mL of 3F4 diluted in 0.1 M NaH₂CO₃, pH 8.9, at 4°C for overnight, washed with TBST, and blocked with 0.1% casein prior to use.

Digestion samples, 150 µL/well of each, were added to 3F4-coated plates for an ELISA assay. The plates were incubated for overnight at 4°C, washed with TBST, and 150 µL of detection antibody POM2AP (0.01 µg/mL) or POM17AP (0.1 µg/mL, in order for the assay sensitivity to be comparable to POM2) was added per well. After incubation of 1 hr at 37°C, plates were washed, substrate (Lumi-Phos Plus) was added and incubated for 30 min at 37°C, and levels of signals were measured with a luminometer as shown in previous examples. Measurement units are defined in relative light units (RLU).

The results of the experiment done with POM2AP are shown in Table 8 and Figure 5, while those of POM 17AP are shown in Table 9 and Figure 6. The calculation of signal/normal ratio for both experiments (POM2AP and POM17AP) is done and shown in Table 10.

**TABLE 8: Detection of PrP^{Sc} Using 3F4 and POM2AP from SHa BH Digested by Trypsin or PK under Various Conditions**

| **Sample** | | **Trypsin (µg/mL)** | | | | | | **PK (µg/mL)** | | | | | |
|---|---|---|---|---|---|---|---|---|---|---|---|---|---|
| | | **0** | **5** | **10** | **20** | **50** | **100** | **0** | **5** | **10** | **20** | **50** | **100** |
| **Normal** | RLU | 2137.7 | 33.2 | 19.7 | 8.5 | 3.4 | 3.4 | 1954.7 | 84.1 | 50.2 | 37.1 | 15.8 | 4.6 |
| | SD | 7.8 | 3.6 | 0.6 | 1.0 | 0.7 | 1.1 | 150.4 | 1.3 | 2.8 | 4.2 | 4.5 | 2.0 |
| **Scrapie-infected** | RLU | **2319.7** | **2058.0** | **1900.3** | **1754.3** | **1544.3** | **1173.3** | **2291.0** | **2310.0** | **2242.0** | **2117.7** | **1154.0** | **79.7** |
| | SD | 23.7 | 13.9 | 20.2 | 26.7 | 84.5 | 48.3 | 39.4 | 29.7 | 22.0 | 28.0 | 242.9 | 7.1 |
| **S/N** | | **1.1** | **62.0** | **96.5** | **205.6** | **447.8** | **346.8** | **1.2** | **27.5** | **44.6** | **57.1** | **73.0** | **17.5** |

| | | | | | | | | | | | | | |
|---|---|---|---|---|---|---|---|---|---|---|---|---|---|
| S/N: signal/normal ratio or Scrapie-infected/Normal ratio. | | | | | | | | | | | | | |

**TABLE 9: Detection of PrP^{Sc} Using 3F4 and POM17AP from SHa BH Digested by Trypsin or PK under Various Conditions**

| **Sample** | | **Trypsin (µg/mL)** | | | | | | **PK (µg/mL)** | | | | | |
|---|---|---|---|---|---|---|---|---|---|---|---|---|---|
| | | **0** | **5** | **10** | **20** | **50** | **100** | **0** | **5** | **10** | **20** | **50** | **100** |
| **Normal** | RLU | 1657.0 | 8.5 | 4.5 | 2.8 | 2.0 | 2.6 | 1676.0 | 11.4 | 7.2 | 5.5 | 3.3 | 2.8 |
| | SD | 32.5 | 2.0 | 1.5 | 0.8 | 0.3 | 0.3 | 17.7 | 1.0 | 0.7 | 1.3 | 0.8 | 0.5 |
| **Scrapie-infected** | RLU | **2544.3** | **2185.3** | **1986.7** | **1576.0** | **812.1** | **345.2** | **3858.7** | **2480.7** | **1638.3** | **1112.0** | **55.9** | **4.7** |
| | SD | 50.3 | 53.1 | 48.6 | 56.8 | 41.7 | 22.8 | 25.3 | 152.5 | 348.1 | 39.6 | 16.0 | 0.7 |
| **S/N** | | **1.5** | **258.3** | **438.6** | **560.3** | **415.0** | **132.1** | **2.3** | **217.3** | **229.1** | **203.4** | **16.8** | **1.7** |

**TABLE 10: Summary of Scrapie-infected Signal to Normal (S/N) Values from Tables 8 and 9.**

| Protease µg/mL | **POM2** | | **POM17** | |
|---|---|---|---|---|
| | Trypsin | PK | Trypsin | PK |
| | S/N | S/N | S/N | S/N |
| 5 | 62 | 28 | 258 | 217 |
| 10 | 97 | 45 | 438 | 229 |
| 20 | 206 | 57 | 560 | 203 |
| 50 | 448 | 73 | 415 | 17 |
| 100 | 347 | 18 | 132 | 2 |

As shown in Table 8 and Figure 5, when detected with POM2AP, normal BH samples untreated with any protease have signals around 2000 RLU due to high content of PrP^{C}. Treatment with 50 µg/mL of trypsin reduced this to background levels of 3 RLU. Similar treatment of scrapie-infected BH samples with 50 µg/mL of trypsin resulted in detection levels of 1544 RLU, with scrapie-infected signal over normal (S/N) of 447 fold; treatment with 100 µg/mL of trypsin yielded a S/N of 346 fold. When treated with 50 µg/mL of PK the S/N was only 73 fold; at 100 µg/mL of PK the scrapie-infected signal was mostly diminished, and the S/N was 17.5 fold.

As shown in Table 9 and Figure 6, when detected with POM17AP (at a 10-fold higher concentration as POM2AP), detection of PrP^{Sc} in scrapie-infected samples was at maximum after treatment with trypsin at 50 µg/mL, indicated by a S/N of 560, while best detection with PK was at 10 µg/mL of PK with S/N of 229. At 100 µg/mL PK the scrapie-infected signal diminished to 4.7 RLU, suggesting full degradation of the protease resistant core of PrP^{Sc}. At similar concentration of trypsin, 100 µg/mL, the RLU was 345.

From the results with either POM2AP or POM17AP as summarized in Table 10, better detection of PrP^{Sc} was observed with trypsin than with PK at any given concentration of proteases, indicated by the higher S/N values of trypsin-digested samples.

When digested with PK and detected with POM2AP, detection level of PrP^{Sc} in scrapie-infected SHa BH was the lowest as indicated by the low S/N values. When the same samples were detected with POM17 the signal was about 4 times higher at optimum concentration of PK. These results are consistent with what is known and expected about the protease resistant properties of PrP^{Sc}; residues 90-231 are resistant to mild protease digestion and therefore can be detected with antibodies with epitope within this region, like POM17, while the N-terminus 23-90 is sensitive and antibodies that bind the octarepeat region, like POM2 and SAF-32, will lose their binding sites. It is also apparent that as we increase the concentration of PK the N-terminus and protease resistant core 90-231 are digested concomitantly. Thus, in order to achieve the goal as proposed in U. S. Patent No. 7,097,997 B1, concentration of PK has to be carefully titrated. As incubation time and temperature will affect enzymatic activity, each study needs be tailored to a specific condition, making it a laborious and unstable process. Another level of complexity is the variations in enzymatic specific activities associated with different preparations, batches and sources of PK.

Treatment with trypsin does not have the same limitations as PK. Antibodies with high avidity like POM2 can be used since the octarepeat region remained intact. While PK cleaves within residues 23-89, trypsin cleaves within residues 23-48 only, leaving residues 49-89, which contains the octarepeat region, available for detection in addition to the protease-resistant core 90-231 (Figures 1 A and 1 B). Thus, we suggest that different detection antibodies can be mixed together and used simultaneously for higher detection sensitivity. For example, POM2 and POM17, when both conjugated with labels such as AP, can be used simultaneously as detection antibodies when another antibody, such as 3F4, is used to capture trypsin-digested PrP^{Sc}.

In addition, PrP^{Sc} seems to be more resistant to trypsin than to PK, because at 100 µg/mL of trypsin S/N values are still over 100. This is consistent with early studies which demonstrated that PK reduced levels of PrP^{Sc} and prion infectivity by several logs while trypsin did not (McKinley et al. (1983) Cell 35:57-62). Of the samples detected with POM2AP, the S/N is 73 for samples treated with 50 µg/mL of PK, while the S/N is 448 for samples treated with the same concentration of trypsin, indicating a 6-fold improvement when using trypsin as compared to using PK. When detected with POM 17AP, an improvement of more than 25 fold (415 vs. 17 RLU) was observed with 50 µg/mL of trypsin, and one of more than 2 fold (560 vs. 203 RLU) was observed with 20 µg/mL of trypsin. Thus, for both antibodies, trypsin treatment gave better detection. It should be noted that although POM2AP was used at 0.01 µg/ml, as compared to POM17AP which was used at 0.1 µg/ml, its detection was as good.

### Example 8

### Trypsin Digests PrP^{Sc} While Preserving Almost Full-length PrP^{Sc}

In order to confirm that trypsin digests PrP^{C} and PrP^{Sc} differently, an immunoblot assay was carried out as described below.

A schematic of the full-length mature PrP sequence, the PK-resistant core of PrP^{Sc}, and the trypsin cleavage site map of PrP is shown in Figure 7A.

Infectious human vCJD and sCJD BH samples were acquired from the National Institute for Biological Standards and Control (NIBSC) CJD Resource Centre in U.K. and correspond to White vCJD (MM), Red sCJD (MM), and Yellow sCJD (MV) strains, respectively. Normal brain homogenate was derived from a patient with vascular encephalopathy from the University of Zurich, corresponding to patient NRPE 327. Twenty five micrograms of normal or infectious human BH sample were digested with 50 µg/ml of trypsin or Proteinase K (PK) in 0.5xTBS with 0.5% Tween20, 0.5% TritonX-100, and 5 mM CaCl₂ for 1 hr at 37°C. Samples were separated by 12% SDS-PAGE in parallel with 10 µg undigested sample for comparison, and immunoblotted with anti-PrP antibodies (3F4, POM2 or POM17). Horseradish peroxidase (HRP)-conjugated goat anti-mouse antibodies were used to detect the Western blots. Chemiluminescent images were acquired via a Kodak Image Station 4000MM. The result is shown in Figure 7B.

In addition, normal or a vCJD human BH sample was digested with increasing concentrations of trypsin and then analyzed by immunoblotting in a similar manner as described above with POM2, 3F4 or POM1 antibody separately. POM1 recognizes an epitope within the protease-resistant core, in particular an epitope within the globular domain of residues about 122-231. The result is shown in Figure 8.

Immunoblot analysis of the digests indicated that trypsin-resistant PrP^{Sc} fragments (expected to be ∼49-231) were larger than PK-resistant fragments (∼90-231) with a substantial fraction of molecules preserved (Figure 7B, 3F4 and POM17 blots). Importantly, mass spectrometry of human recombinant PrP₂₃₋₂₃₁ validated that trypsin could cleave at all the sites predicted by the PrP sequence, thus confirming that the tryptic sites were truly conformationally protected in PrP^{Sc} (data not shown). Further examination revealed that digestion with trypsin but not PK preserved the PrP octapeptide repeats recognized by the POM2 antibody (Figure 7B, POM2 blots).

By contrast, PrP^{C} was digested by both proteases, and was no longer detected by POM2 (residues ∼59-89) or 3F4 (residues ∼109-112) (Figure 7B and Figure 8, Normal sample lanes). However, when we immunoblotted the PrP^{C} digests with POM17 or POM1 (against helix one, residues ∼144-155), we observed a trypsin-resistant fragment of ∼25 kDa. Given that the structure of PrP^{C} is composed of a globular domain (∼122-231) following an unstructured amino-terminus (Zahn, R., et al (2000) Proc Natl Acad Sci U S A 97:145-150), the 25 kDa tryptic fragment likely included the globular domain as it was the appropriate molecular weight and detected by POM I and POM 17 but not POM2 or 3F4 (Fig. 7B, 8). Interestingly, this would suggest that the globular domain is tightly folded and that the trypsin cleavage sites within the POM17/POM1 epitope (R148 and R151) are not available for cleavage, contrary to how they appear in the solution structure (Zahn, R., et al (2000) Proc Natl Acad Sci U S A 97:145-150). To better understand the resistance of the domain to digestion, we digested both normal and vCJD BHs with increasing amounts of trypsin. We found the amino-terminal sequences of PrP^{C} extremely labile since no PrP was detected by either POM2 or 3F4 at any of the trypsin concentrations tested, but the PrP^{C} globular domain and PrP^{Sc} from vCJD BH were resistant to digestion (Figure 8, POM1). Additionally, the migration patterns of trypsin-digested PrP^{Sc} and PrP^{C} differed widely, so we could distinguish PrP^{C} and PrP^{Sc} trypsin-resistant fragments. Therefore, we conclude that trypsin digestion is a viable method of separating PrP^{C} from PrP^{Sc}.

### Example 9

### Measuring the Stability of CJD Strains Against Trypsin Digestion

PK has commonly been used to characterize strain-dependent diversity in PrP^{Sc} conformations. These differences are manifested as susceptibility to protease digestion and alterations in amino-terminal cleavage sites, as observed for types 1 and 2 sCJD. Digestion with trypsin, however, has the unique advantage that cleavage sites are known, with several sites lying within well-characterized antibody epitopes (Figure 9A). Therefore, the sensitivity of specific PrP sequences to proteolytic cleavage could be assessed. As such, we tried to detect remaining PrP sequences after digestion with trypsin or PK for normal (129 MM), vCJD (129 MM), and sporadic CJD (sCJD) (129 MM and 129MV) BHs with increasing amounts of protease.

1% BH (∼1 mg/ml total protein) diluted in TBS with 2% Sarkosyl (TBSS) was digested with 0, 1, 10, or 100 µg/ml trypsin or PK for 1 hr at 37°C. Digestions were stopped with the addition of 2 mM PMSF and a Complete Mini protease inhibitor cocktail (Roche, Indianapolis, IN) in four volumes of TBS. The samples were then detected by direct ELISA. Approximately 500 nl of digested 10% BH was centrifuged at 14,000 rpm for 30 minutes at 4°C. The PrP^{Sc} pellets were resuspended in 6M GdnSCN, diluted with an equal volume of 0.1M NaHCO3 pH8.9, and passively coated to ELISA plates overnight. The plates were blocked in 0.1 x BlockerCasein in TBS (Pierce) and coated PrP was detected via 0.1 µg/ml 3F4, POM2 or POM17 antibodies and alkaline phosphatase (AP)-conjugated goat anti-mouse antibodies (Pierce). All samples were analyzed by ELISA in triplicate and were washed six times with TBS 0.05% Tween20 between antibody incubations. Finally, LumiphosPlus substrate (Lumigen, Southfield, MI) with 0.05% SDS was added to the wells and incubated for 30 minutes at 37°C before the luminescence was measured via a Lumiskan luminometer (Thermo Electron Corporation, Waltham, MA). The raw data for normal and vCJD BH samples are shown in Figure 9B, whereas normalized data for vCJD MM, sCJD MM, and sCJD MV (Infectious - Normal, RLU) are shown in Figure 9C.

Of note, very little PrP^{C}-derived signal was present in the pellets of undigested samples in both normal and infectious BHs, which completely disappeared with only 1 µg/ml protease (Figure 9B). Furthermore, the -25 kDa trypsin-resistant fragment of PrP^{C} was not detected in the pellet, suggesting that the domain remained in solution during centrifugation (Figure 9B, POM 17).

When examining PrP^{Sc} from a vCJD BH, we found significant differences in the exposure of epitopes to trypsin versus PK. While the 3F4 epitope was preserved at the same rate when either protease was utilized (Figure 9B, 3F4), the POM2 epitope showed dramatically different results. The octarepeats were excised only at high concentrations of trypsin but rapidly disappeared with PK digestion (Figure 9B, POM2). To directly compare the amount of PrP^{Sc} preserved by each protease, we subtracted the PrP^{C}-derived signal detected in normal BH from each of the infectious samples to obtain the normalized signal (Figure 9C, Infectious - Normal). As shown in the figure, the amount of normalized signal detected by POM2 in vCJD or sCJD after digestion with 100 µg/ml of trypsin is higher or about the same level as the amount detected in the same CJD after digestion with only 1 µg/ml of PK. By this comparison, trypsin digestion clearly preserved more POM2-detected PrP^{Sc} than PK since there are no trypsin cleavage sites within the octarepeat region.

Interestingly, POM17 detection of the PrP^{Sc} core particle revealed that the vast majority of helix one, the region containing the epitope recognized by POM 17, was resistant at every concentration of trypsin tested (Figure 9C, POM17), suggesting that R148 and R151 are conformationally protected. PK, however, digested POM17-detected PrP^{Sc} with increasing concentrations of protease, in agreement with previous reports (see Aguzzi and Polymenidou, (2004) Cell 116: 313-327).

Taken together with the immunoblot analysis results shown in Example 8, these results suggest that trypsin digestion of PrP^{Sc}, especially at higher concentrations of protease, generates two populations of trypsin-resistant fragments, one population being the expected long fragments spanning residues of ∼49-231, the other population spanning residues of ∼111-231, lacking the 3F4 epitope and octarepeats. In conclusion, we find that the 3F4 epitope is equally exposed to cleavage by trypsin or PK, but that other sequences such as the octarepeat and helix one regions are uniquely preserved in trypsin digestion, making trypsin an ideal tool for analysis of PrP^{Sc}.

### Example 10

### Trypsin and PK Digestion of Various Human CJD Strain Brain Homogenate Samples and Detection with POM and 3F4 Antibodies

In this example, an experiment similar to the one described in Example 7 was carried out on several human BH samples instead of Syrian Hamster BH samples. Normal or various CJD-infected human BH samples, including vCJD (M/M), sCJD (M/M1) and sCJD (M/V2), were digested with increasing concentrations of trypsin or PK and detected by Sandwich ELISA. In order to see whether the preservation of the octarepeat region by trypsin digestion can enhance PrP detection, a detection antibody against the octarepeat region, POM2AP, was used at the same concentration as and compared to another detection antibody which is against a non-octarepeat region, POM17AP. In Example 7, POM2AP was used at one tenth of the concentration of POM17AP.

10% BH diluted 10-fold into TBS with 2% Sarkosyl (TBSS) was digested with 0, 1, 10, or 100 µg/ml trypsin or PK for 1 hr at 37°C. Digestions were stopped by adding 2 mM PMSF and Complete Mini protease inhibitor cocktail (Roche, Indianapolis, IN) in four volumes of TBS. The samples were then detected by Sandwich ELISA. Two hundred and fifty nl of digested 10% BH was denatured with 0. 1 M NaOH for 10 minutes at 25°C, neutralized with NaH₂PO₄ pH 4.3, and PrP was captured with 3F4-coated plates (375 ng/well) and detected with 0.02 µg/ml AP-conjugated POM2 or POM 17. All samples were analyzed by ELISA in triplicate and were washed six times with TBS 0.05% Tween20 between antibody incubations. Finally, LumiphosPlus substrate (Lumigen, Southfield, MI) with 0.05% SDS was added to the wells and incubated for 30 minutes at 37°C before the luminescence was measured via a Lumiskan luminometer (Thermo Electron Corporation, Waltham, MA). The raw data for normal and various CJD BH samples are shown in Figure 10, whereas the estimated PrP^{Sc} amounts for vCJD (M/M) (vCJD - Normal, RLU) are shown in Figure 11.

As shown in Figures 10 and 11, at the trypsin digestion concentration of 10 µg/ml in TBSS, PrP^{C} in the normal BH sample could not be detected with POM2AP or POM17AP when captured with 3F4, while PrP^{Sc} in the CJD BH samples had significant amounts of the long trypsin-resistant fragments left which could be detected with POM2AP or POM 17AP when captured with 3F4. PK digestion, however, did not preserve the detection of the octarepeat region by POM2AP when the PK concentration was high enough to eliminate the background signal generated from PrP^{C}. For both vCJD and sCJD strains, POM17AP-detected PrP amount was comparable for trypsin or PK-digested samples, although trypsin preserved more epitopes at certain protease concentrations. By contrast, POM2AP-detected PrP^{Sc} rapidly disappeared with increasing amounts of PK in the digestion, but decreased much more slowly with increasing amounts of trypsin. Furthermore, at the same detection concentration of 0.02 µg/ml, POM2AP generated about 10 times the signal (RLU) as that generated by POM17AP in BH samples of all three CJD strains digested with various concentrations of trypsin, a result that is attributable to the avidity of the POM2AP antibody for the repeated octapeptide sequence. In conclusion, preservation of the octarepeat region significantly enhanced PrP^{Sc} detection.

## Claims

1. An assay method, comprising the steps of:
(a) obtaining a sample suspected of containing a pathogenic form of prion protein (PrP^{Sc}) which has a protease-resistant core and an octarepeat region, wherein said sample may or may not contain a normal form of prion protein (PrP^{C});
(b) treating said sample with at least one site-specific protease under conditions in which proteolytic digestion of said PrP^{Sc} and said PrP^{C}, if present, results in said PrP^{Sc} and said PrP^{C}, if present, being cleaved in at least 90% of all available protease cleavage sites, wherein said site-specific protease is trypsin or S-V8,
i. wherein said PrP^{Sc} has no cleavage site for said protease within said octarepeat region or between said octarepeat region and said protease-resistant core whereby a fragment of amino-proximal region including said octarepeat region remains connected to said protease-resistant core after said proteolytic digestion, and
ii. wherein said PrP^{C} has at least one available cleavage site for said protease within amino acid region corresponding to said protease-resistant core, and said at least one available cleavage site is cleaved by said proteolytic digestion;
(c) preventing any further proteolytic digestion following said proteolytic digestion in (b) by adding a protease inhibitor or removing said protease;
(d) denaturing said site-specific-protease-treated PrP^{Sc} thereby providing denatured PrP^{Sc}; and
(e) detecting the presence of said denatured PrP^{Sc} using at least two binding partners, a first binding partner and a second binding partner,
i. wherein said first binding partner specifically binds a first epitope which is located within said fragment of amino-proximal region that remains connected to said protease-resistant core after said proteolytic digestion, and
ii. wherein said second binding partner specifically binds a second epitope which is located within said protease-resistant core, wherein said second epitope is in a region of said PrP^{C} that is separated from said first epitope after said proteolytic digestion.

2. The method of claim 1,
(a) wherein said PrP^{Sc} comprises a sequence selected from SEQ ID NOs. 1 to 10; or
(b) wherein said first epitope is within said octarepeat region; wherein optionally said first binding partner is a monoclonal antibody selected from the group consisting of POM2, POM11, POM12, POM14, 3B5, 4F2, 13F10, SAF-15, SAF-31, SAF-32, SAF-33, SAF-34, SAF-35, and SAF-37; or
(c) wherein said first epitope is outside of said octarepeat region; wherein optionally said first binding partner is a monoclonal antibody selected from the group consisting of BAR210, BAR231, and 14D3.

3. The method of any one of claims 1-2,
(a) wherein said site-specific protease is trypsin; or
(b) wherein said site-specific protease is S-V8; wherein optionally said second binding partner is a monoclonal antibody selected from the group consisting of SAF-53, SAF-54, SAF-60, SAF-61, SAF-66, SAF-69, SAF-70, SAF-75, SAF-76, Pri917, BAR234, Sha31, 11B9, 12F10, 6H4, and POM5; or
(c) wherein said binding partners are aptamers; or
(d) wherein said binding partners are antibodies; wherein optionally said antibodies are monoclonal antibodies; or
(e) wherein said detecting step is carried out using an ELISA.

4. The method of claim 3(e),
(a) wherein said first binding partner is a capture binding partner to capture prion proteins, and wherein said second binding partner is a detection binding partner to detect said PrP^{Sc}; or
(b) wherein said second binding partner is a capture binding partner to capture prion proteins, and wherein said first binding partner is a detection binding partner to detect said PrP^{Sc}.

5. The method of claim 4(a) or 4(b), wherein said detection binding partner is labeled; wherein optionally said detection binding partner is labeled with an alkaline phosphatase conjugate.

6. The method of claim 3(a),
(a) wherein said second binding partner is a monoclonal antibody selected from the group consisting of 3F4, POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D 18, 6H4, and BDI115; or
(b) wherein said PrP^{C} contains a globular domain within amino acid sequences corresponding to said protease-resistant core, and wherein said second epitope is located within said globular domain.

7. The method of claim 6(b), wherein said second binding partner is a monoclonal antibody selected from the group consisting of POM1, POM4, POM5, POM6, POM7, POM8, POM9, POM10, POM 13, POM15, POM 16, POM17, POM19, SAF-2, SAF-4, SAF-8, SAF-9, SAF-10, SAF-12, SAF-13, SAF-14, SAF-22, SAF24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, and BDI115.

8. The method of claim 1 or 2(a),
(a) wherein said site-specific protease is immobilized; or
(b) wherein said protease inhibitor is phenylmethylsulphonyl fluoride; or
(c) wherein said denaturing step comprises treating said sample with a guanidinium compound; and optionally further comprising a step of diluting said sample following said denaturing step; or
(d) wherein said denaturing step is carried out by exposing said sample to high pH or low pH; and optionally further comprising the step of neutralizing said high pH or said low pH following said denaturing step; or
(e) wherein said sample is obtained from a living or once-living organism; wherein optionally said living or once-living organism is selected from the group consisting of human, monkey, hamster, bovine, sheep, mouse, elk, and deer; or
(f) wherein said sample is derived from the group consisting of food supply, whole blood, blood products, blood fractions, blood components, plasma, platelets, serum, cerebrospinal fluid, organs, cells, brain tissue, nervous system tissue, muscle tissue, fatty tissue, bone marrow, urine, tears, non-nervous system tissue, biopsies, necropsies, and contaminated instruments; or
(g) wherein said sample is derived from the group consisting of whole blood, blood products, blood fractions, blood components, plasma, platelets, red blood cells, and serum; or
(h) wherein said sample is obtained from a biopsy, autopsy or necropsy.

9. A kit for detecting the presence of a pathogenic form of prion protein (PrP^{Sc}) which has a protease-resistant core and an octarepeat region in a sample suspected of containing said PrP^{Sc}, wherein said sample may or may not contain a normal form of prion protein (PrP^{C}), the kit comprising:
(a) at least one site-specific protease, wherein said site-specific protease is trypsin or S-V8,
i. wherein said PrP^{Sc} has no cleavage site for said protease within said octarepeat region or between said octarepeat region and said protease-resistant core whereby a fragment of amino-proximal region including said octarepeat region remains connected to said protease-resistant core after a proteolytic digestion of PrP^{Sc} by said protease which results in said PrP^{Sc} being cleaved in at least 90% of all available protease cleavage sites, and
ii. wherein said PrP^{C} has at least one available cleavage site for said protease within amino acid region corresponding to said protease-resistant core;
(b) optionally, a protease inhibitor which is capable of inhibiting the activity of said protease;
(c) optionally, a denaturant which is capable of denaturing said PrP^{Sc};
(d) at least two binding partners, a first binding partner and a second binding partner,
i. wherein said first binding partner specifically binds a first epitope which is located within said fragment of amino-proximal region that remains connected to said protease-resistant core after a proteolytic digestion of said PrP^{Sc} which results in said PrP^{Sc} being cleaved in at least 90% of all available protease cleavage sites by said protease, and
ii. wherein said second binding partner specifically binds a second epitope which is located within said protease-resistant core, wherein said second epitope is in a region of said PrP^{C} that is separated from said first epitope after a proteolytic digestion of said PrP^{C} which results in said PrP^{C} being cleaved in at least 90% of all available protease cleavage sites by said protease; and
(e) an instruction for using said kit to detect the presence of any pathogenic form of prion protein.

10. The kit of claim 9, wherein said PrP^{Sc} comprises a sequence selected from SEQ ID NOs. 1 to 10.

11. The kit of claim 9 or 10,
(a) wherein said first epitope is within said octarepeat region; wherein optionally said first binding partner is a monoclonal antibody selected from the group consisting of POM2, POM11, POM12, POM14, 3B5, 4F2, 13F10, SAF-15, SAF-31, SAF-32, SAF-33, SAF-34, SAF-35, and SAF-37; or
(b) wherein said first epitope is outside of said octarepeat region; wherein optionally said first binding partner is a monoclonal antibody selected from the group consisting of BAR210, BAR231, and 14D3; or
(c) wherein said site-specific protease is trypsin; or
(d) wherein said site-specific protease is S-V8; wherein optionally said second binding partner is a monoclonal antibody selected from the group consisting of SAF-53, SAF-54, SAF-60, SAF-61, SAF-66, SAF-69, SAF-70, SAF-75, SAF-76, Pri917, BAR234, Sha31, 11B9, 12F10, 6H4, and POM5; or
(e) wherein said binding partners are aptamers; or
(f) wherein said binding partners are antibodies; wherein optionally said antibodies are monoclonal antibodies; or
(g) wherein said kit comprises an ELISA kit.

12. The kit of claim 11(g)
(a) wherein said first binding partner is a capture binding partner to capture prion proteins, and wherein said second binding partner is a detection binding partner to detect said PrP^{Sc}; or
(b) wherein said second binding partner is as a capture binding partner to capture prion proteins, and wherein said first binding partner is as a detection binding partner to detect said PrP^{Sc}.

13. The kit of claim 12(a) or 12(b), wherein said detection binding partner is labeled; wherein optionally said detection binding partner is labeled with an alkaline phosphatase conjugate.

14. The kit of claim 11(c),
(a) wherein said second binding partner is a monoclonal antibody selected from the group consisting of 3F4, POM1, POM4, POM5, POM6, POM7, POM8, POM9, POM10, POM 13, POM15, POM 16, POM 17, POM 19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4 and BDI115; or
(b) wherein said PrP^{C} contains a globular domain within amino acid sequences corresponding to said protease-resistant core, and wherein said second epitope is located within said globular domain; wherein optionally said second binding partner is a monoclonal antibody selected from the group consisting of POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, and BDI115.

15. The kit of claim 9 or 10,
(a) wherein said site-specific protease is immobilized; or
(b) wherein said optional protease inhibitor is phenylmethylsulphonyl fluoride; or
(c) wherein said optional denaturant is a guanidinium compound; or
(d) wherein said optional denaturant is a basic reagent or an acidic reagent.

## Patentansprüche

1. Assayverfahren, bei dem man in den folgenden Schritten:
(a) eine Probe erhält, von der vermutet wird, dass sie eine pathogene Form des Prion-Proteins (PrP^{Sc}) enthält, die einen proteaseresistenten Kern und eine Oktarepeat-Region besitzt, wobei die Probe eine normale Form des Prion-Proteins (PrP^{c}) enthalten kann oder nicht;
(b) die Probe mit mindestens einer stellenspezifischen Protease unter Bedingungen behandelt, unter denen der proteolytische Verdau von PrP^{Sc} und PrP^{c}, wenn vorhanden, dazu führt, dass PrP^{Sc} und PrP^{c}, wenn vorhanden, an mindestens 90% aller vorhandener Protease-Spaltstellen gespalten werden, wobei die stellenspezifische Protease Trypsin oder S-V8 ist,
i. wobei das PrP^{Sc} keine Spaltstelle für die Protease innerhalb der Oktarepeat-Region oder zwischen der Oktarepeat-Region und dem proteaseresistenten Kern besitzt, wobei ein Fragment der aminoproximalen Region, das die Oktarepeat-Region enthält, mit dem proteaseresistenten Kern nach dem proteolytischen Verdau verbunden bleibt, und
ii. wobei das PrP^{c} mindestens eine verfügbare Spaltstelle für die Protease innerhalb der Aminosäureregion hat, die dem proteaseresistenten Kern entspricht, und die mindestens eine verfügbare Spaltstelle durch den proteolytischen Verdau gespalten wird;
(c) jeglichen weiteren proteolytischen Verdau nach dem proteolytischen Verdau unter (b) durch Zugabe eines Protease-Inhibitors oder durch Entfernen der Protease verhindert,
(d) das mit der stellenspezifischen Protease behandelte PrP^{Sc} denaturiert, wodurch denaturiertes PrP^{Sc} bereitgestellt wird, und
(e) das Vorliegen des denaturierten PrP^{Sc} unter Verwendung von mindestens zwei Bindungspartnern, eines ersten Bindungspartners und eines zweiten Bindungspartners, nachweist,
i. wobei der erste Bindungspartner spezifisch an ein erstes Epitop bindet, das sich innerhalb des Fragments der aminoproximalen Region befindet, das mit dem proteaseresistenten Kern nach dem proteolytischen Verdau verbunden bleibt, und
ii. wobei der zweite Bindungspartner spezifisch an ein zweites Epitop bindet, das sich innerhalb des proteaseresistenten Kerns befindet, wobei das zweite Epitop in einer Region von PrP^{c} liegt, die von dem ersten Epitop nach der proteolytischen Verdau abgetrennt ist.

2. Verfahren nach Anspruch 1,
(a) wobei das PrP^{Sc} eine Sequenz umfasst, die aus den SEQ ID NR. 1 bis 10 ausgewählt ist;
(b) wobei das erste Epitop innerhalb der Oktarepeat-Region liegt, wobei gegebenenfalls der erste Bindungspartner ein monoklonaler Antikörper ist, der aus der Gruppe ausgewählt wird, bestehend aus POM2, POM11, POM12, POM14, 3B5, 4F2, 13F10, SAF-15, SAF-31, SAF-32, SAF-33, SAF-34, SAF-35 und SAF-37, oder
(c) wobei sich das erste Epitop außerhalb der Oktarepeat-Region befindet, wobei gegebenenfalls der erste Bindungspartner ein monoklonaler Antikörper ist, der aus der Gruppe ausgewählt wird, bestehend aus BAR210, BAR231 und 14D3.

3. Verfahren nach einem der Ansprüche 1-2,
(a) wobei die stellenspezifische Protease Trypsin ist oder
(b) wobei die stellenspezifische Protease S-V8 ist, wobei gegebenenfalls der zweite Bindungspartner ein monoklonaler Antikörper ist, der aus der Gruppe ausgewählt wird, bestehend aus SAF-53, SAF-54, SAF-60, SAF-61, SAF-66, SAF-69, SAF-70, SAF-75, SAF-76, Pri917, BAR234, Sha31, 11B9, 12F10, 6H4 und POM5, oder
(c) wobei die Bindungspartner Aptamere sind oder
(d) wobei die Bindungspartner Antikörper sind, wobei die Antikörper gegebenenfalls monoklonale Antikörper sind, oder
(e) wobei der Nachweisschritt unter Verwendung eines ELISA durchgeführt wird.

4. Verfahren nach Anspruch 3(e),
(a) wobei der erste Bindungspartner ein Capture-Bindungspartner zum Einfangen von Prion-Proteinen ist und wobei der zweite Bindungspartner ein Nachweis-Bindungspartner zum Nachweis von PrP^{Sc} ist,
(b) wobei der zweite Bindungspartner ein Capture-Bindungspartner zum Einfangen von Prion-Proteinen ist und wobei der erste Bindungspartner ein Nachweis-Bindungspartner zum Nachweis von PrP^{Sc} ist.

5. Verfahren nach Anspruch 4(a) oder 4(b), wobei der Nachweis-Bindungspartner markiert ist, wobei gegebenenfalls der Nachweis-Bindungspartner mit einem alkalische Phosphatase-Konjugat markiert ist.

6. Verfahren nach Anspruch 3(a),
(a) wobei der zweite Bindungspartner ein monoklonaler Antikörper ist, der aus der Gruppe ausgewählt wird, bestehend aus 3F4, POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4 und BDI115, oder
(c) wobei das PrP^{c} eine globuläre Domäne innerhalb von Aminosäuresequenzen enthält, die dem proteaseresistenten Kern entsprechen, und wobei sich das zweite Epitop innerhalb der globulären Domäne befindet.

7. Verfahren nach Anspruch 6(b), wobei der zweite Bindungspartner ein monoklonaler Antikörper ist, der aus der Gruppe ausgewählt wird, bestehend aus POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4 und BDI115.

8. Verfahren nach Anspruch 1 oder 2(a),
(a) wobei die stellenspezifische Protease immobilisiert ist oder
(b) wobei der Protease-Inhibitor Phenylmethylsulfonylfluorid ist oder
(c) wobei man in dem Denaturierungsschritt die Probe mit einer Guanidinium-Verbindung behandelt, und das gegebenenfalls weiterhin einen Schritt umfasst, in dem man die Probe nach dem Denaturierungsschritt verdünnt, oder
(d) wobei der Denaturierungsschritt erfolgt, indem man die Probe einem hohen pH-Wert oder einem niedrigen pH-Wert aussetzt, und das gegebenenfalls ferner den Schritt umfasst, in dem man den hohen pH-Wert oder den niedrigen pH-Wert nach dem Denaturierungsschritt neutralisiert, oder
(e) wobei die Probe aus einem lebenden oder ehemals lebenden Organismus erhalten wird, wobei der lebende oder ehemals lebende Organismus gegebenenfalls aus der Gruppe ausgewählt wird, bestehend aus Mensch, Affe, Hamster, Rind, Schaf, Maus, Elch und Hirsch, oder
(f) wobei die Probe aus der Gruppe stammt, bestehend aus Nahrung, Vollblut, Blutprodukten, Blutfraktionen, Blutbestandteilen, Plasma, Blutplättchen, Serum, Zerebrospinalflüssigkeit, Organen, Zellen, Hirngewebe, Nervensystemgewebe, Muskelgewebe, Fettgewebe, Knochenmark, Urin, Tränen, Nicht-Nervensystemgewebe, Biopsien, Nekropsien und kontaminierten Instrumenten, oder
(g) wobei die Probe aus der Gruppe stammt, bestehend aus Vollblut, Blutprodukten, Blutfraktionen, Blutbestandteilen, Plasma, Blutplättchen, roten Blutzellen und Serum, oder
(h) wobei die Probe aus einer Biopsie, Autopsie oder Nekropsie erhalten wird.

9. Kit zum Nachweis des Vorliegens einer pathogenen Form des Prion-Proteins (PrP^{Sc}), die einen proteaseresistenten Kern und eine Oktarepeat-Region besitzt, in einer Probe, die im Verdacht steht, dass sie das PrP^{Sc} enthält, wobei die Probe eine normale Form des Prion-Proteins (PrP^{c}) enthalten kann oder nicht, wobei das Kit Folgendes umfasst:
(a) mindestens eine stellenspezifische Protease, wobei die stellenspezifische Protease Trypsin oder S-V8 ist,
i. wobei das PrP^{Sc} keine Spaltstelle für die Protease innerhalb der Oktarepeat-Region oder zwischen der Oktarepeat-Region und dem proteaseresistenten Kern besitzt, wobei ein Fragment der aminoproximalen Region, das die Oktarepeat-Region enthält, mit dem proteaseresistenten Kern nach dem proteolytischen Verdau von PrP^{Sc} durch die Protease verbunden bleibt, was dazu führt, dass das PrP^{Sc} an mindestens 90% aller vorhandener Protease-Spaltstellen gespalten wird, und
ii. wobei das PrP^{c} mindestens eine verfügbare Spaltstelle für die Protease innerhalb einer Aminosäureregion besitzt, die dem proteaseresistenten Kern entspricht,
(b) gegebenenfalls einen Protease-Inhibitor, der die Aktivität der Protease hemmen kann,
(c) gegebenenfalls ein Denaturierungsmittel, das das PrP^{Sc} denaturieren kann,
(d) mindestens zwei Bindungspartner, einen ersten Bindungspartner und einen zweiten Bindungspartner,
i. wobei der erste Bindungspartner spezifisch an ein erstes Epitop bindet, das sich innerhalb des Fragments der aminoproximalen Region befindet, die mit dem proteaseresistenten Kern nach einem proteolytischen Verdau des PrP^{Sc} verbunden bleibt, was dazu führt, dass das PrP^{Sc} an mindestens 90% aller vorhandener Protease-Spaltstellen durch die Protease gespalten wird, und
ii. wobei der zweite Bindungspartner spezifisch an ein zweites Epitop bindet, das sich innerhalb des proteaseresistenten Kerns befindet, wobei das zweite Epitop in einer Region von PrP^{c} liegt, die von dem ersten Epitop nach einem proteolytischen Verdau des PrP^{c} abgetrennt ist, was dazu führt, dass das PrP^{c} an mindestens 90% aller vorhandener Protease-Spaltstellen durch die Protease gespalten wird, und
(e) eine Anleitung für die Verwendung des Kits zum Nachweis des Vorliegens einer pathogenen Form des Prion-Proteins.

10. Kit nach Anspruch 9, wobei das PrP^{Sc} eine Sequenz umfasst, die aus den SEQ ID NR. 1 bis 10 ausgewählt ist.

11. Kit nach Anspruch 9 oder 10,
(a) wobei das erste Epitop innerhalb der Oktarepeat-Region liegt, wobei gegebenenfalls der erste Bindungspartner ein monoklonaler Antikörper ist, der aus der Gruppe ausgewählt wird, bestehend aus POM2, POM11, POM12, POM14, 3B5, 4F2, 13F10, SAF-15, SAF-31, SAF-32, SAF-33, SAF-34, SAF-35 und SAF-37, oder
(b) wobei das erste Epitop sich außerhalb der Oktarepeat-Region befindet, wobei gegebenenfalls der erste Bindungspartner ein monoklonaler Antikörper ist, der aus der Gruppe ausgewählt wird, bestehend aus BAR210, BAR231 und 14D3, oder
(c) wobei die stellenspezifische Protease Trypsin ist oder
(d) wobei die stellenspezifische Protease S-V8 ist, wobei gegebenenfalls der zweite Bindungspartner ein monoklonaler Antikörper ist, der aus der Gruppe ausgewählt wird, bestehend aus SAF-53, SAF-54, SAF-60, SAF-61, SAF-66, SAF-69, SAF-70, SAF-75, SAF-76, Pri917, BAR234, Sha31, 11B9, 12F10, 6H4 und POM5, oder
(e) wobei die Bindungspartner Aptamere sind oder
(f) wobei die Bindungspartner Antikörper sind, wobei die Antikörper gegebenenfalls monoklonale Antikörper sind, oder
(g) wobei das Kit ein ELISA-Kit umfasst.

12. Kit nach Anspruch 11(g),
(a) wobei der erste Bindungspartner ein Capture-Bindungspartner zum Einfangen von Prion-Proteinen ist und wobei der zweite Bindungspartner ein Nachweis-Bindungspartner zum Nachweis von PrP^{Sc} ist,
(b) wobei der zweite Bindungspartner ein Capture-Bindungspartner zum Einfangen von Prion-Proteinen ist und wobei der erste Bindungspartner ein Nachweis-Bindungspartner zum Nachweis von PrP^{Sc} ist.

13. Kit nach Anspruch 12(a) oder 12(b), wobei der Nachweis-Bindungspartner markiert ist, wobei der Nachweis-Bindungspartner gegebenenfalls mit einem alkalische Phosphatase-Konjugat markiert ist.

14. Kit nach Anspruch 11(c),
(a) wobei der zweite Bindungspartner ein monoklonaler Antikörper ist, der aus der Gruppe ausgewählt wird, bestehend aus 3F4, POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4 und BDI115, oder
(b) wobei das PrP^{c} eine globuläre Domäne innerhalb von Aminosäuresequenzen enthält, die dem proteaseresistenten Kern entsprechen, und wobei sich das zweite Epitop innerhalb dieser globulären Domäne befindet, wobei gegebenenfalls der zweite Bindungspartner ein monoklonaler Antikörper ist, der aus der Gruppe ausgewählt wird, bestehend aus POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4 und BDI115.

15. Kit nach Anspruch 9 oder 10,
(a) wobei die stellenspezifische Protease immobilisiert ist oder
(b) wobei der optionale Protease-Inhibitor Phenylmethylsulfonylfluorid ist,
(c) wobei das optionale Denaturierungsmittel eine Guanidinium-Verbindung ist,
(d) wobei das optionale Denaturierungsmittel ein basisches Reagenz oder ein saures Reagenz ist.

## Revendications

1. Méthode d'analyse comprenant les étapes de :
(a) obtention d'un échantillon que l'on suspecte de contenir une forme pathogène de la protéine prion (PrP^{Sc}), qui a un noyau résistant aux protéases et une région à huit répétitions, ledit échantillon pouvant, ou non, contenir une forme normale de la protéine prion (PrP^{c}) ;
(b) traitement dudit échantillon avec au moins une protéase spécifique de site, dans des conditions dans lesquelles la digestion protéolytique dudit PrP^{Sc} et dudit PrP^{c}, s'ils sont présents, conduit au clivage dudit PrP^{Sc} et dudit PrP^{c}, s'ils sont présents, sur au moins 90 % de l'ensemble des sites disponibles de clivage par des protéases, ladite protéase spécifique de site étant la trypsine ou le S-V8,
i. ledit PrP^{Sc} n'ayant pas de site de clivage pour ladite protéase à l'intérieur de ladite région à huit répétitions ou entre ladite région à huit répétitions et ledit noyau résistant aux protéases, de sorte qu'un fragment de la région amino-proximale comprenant ladite région à huit répétitions va rester connecté audit noyau résistant aux protéases après ladite digestion protéolytique, et
ii.ledit PrP^{c} ayant au moins un site de clivage disponible pour ladite protéase à l'intérieur de la région d'acides aminés correspondant audit noyau résistant aux protéases, ledit ou lesdits sites de clivage disponibles étant clivés par ladite digestion protéolytique ;
(c) empêchement de toute digestion protéolytique plus poussée suivant ladite digestion protéolytique de (b), par addition d'un inhibiteur de protéase ou par élimination de ladite protéase ;
(d) dénaturation dudit PrP^{Sc} traité par une protéase spécifique de site, de façon à donner un PrP^{Sc} dénaturé ; et
(e) détection de la présence dudit PrP^{Sc} dénaturé par utilisation d'au moins deux partenaires de liaison, un premier partenaire de liaison et un deuxième partenaire de liaison,
i. ledit premier partenaire de liaison se liant d'une manière spécifique à un premier épitope qui est situé à l'intérieur du fragment de la région amino-proximale qui reste connecté audit noyau résistant aux protéases après ladite digestion protéolytique, et
ii.ledit deuxième partenaire de liaison se liant d'une manière spécifique à un deuxième épitope qui est situé à l'intérieur dudit noyau résistant aux protéases, ledit deuxième épitope se trouvant dans une région dudit PrP^{c} qui est séparée dudit premier épitope après ladite digestion protéolytique.

2. Procédé de la revendication 1,
(a) dans lequel ledit PrP^{Sc} comprend une séquence choisie parmi SEQ ID N° 1 à 10 ;
(b) dans lequel ledit premier épitope se trouve à l'intérieur de ladite région à huit répétitions ; ledit premier partenaire de liaison étant en option un anticorps monoclonal choisi dans le groupe consistant en POM2, POM11, POM12, POM14, 3B5, 4F2, 13F10, SAF-15, SAF-31, SAF-32, SAF-33, SAF-34, SAF-35 et SAF-37 ; ou
(c) dans lequel ledit premier épitope se trouve à l'extérieur de ladite région à huit répétitions ; ledit premier partenaire de liaison étant en option un anticorps monoclonal choisi dans le groupe consistant en BAR210, BAR231 et 14D3.

3. Procédé de l'une quelconque des revendications 1-2,
(a) dans lequel ladite protéase spécifique de site est la trypsine ; ou
(b) dans lequel ladite protéase spécifique de site est le S-V8 ; ledit deuxième partenaire de liaison étant en option un anticorps monoclonal choisi dans le groupe consistant en SAF-53, SAF-54, SAF-60, SAF-61, SAF-66, SAF-69, SAF-70, SAF-75, SAF-76, Pri917, BAR234, Sha31, 11B9, 12F10, 6H4 et POM5 ; ou
(c) dans lequel lesdits partenaires de liaison sont des aptamères ; ou
(d) dans lequel lesdits partenaires de liaison sont des anticorps ; lesdits anticorps étant en option des anticorps monoclonaux ; ou
(e) dans lequel ladite étape de détection est mise en oeuvre par utilisation d'une ELISA.

4. Procédé de la revendication 3(e),
(a) dans lequel ledit premier partenaire de liaison est un partenaire de liaison par capture, pour capturer les protéines prions, ledit deuxième partenaire de liaison étant un partenaire de liaison par détection, pour détecter ledit PrP^{Sc} ; ou
(b) dans lequel ledit deuxième partenaire de liaison est un partenaire de liaison par capture pour capturer les protéines prions, et ledit premier partenaire de liaison étant un partenaire de liaison par détection pour détecter ledit PrP^{Sc}.

5. Procédé de la revendication 4 (a) ou 4(b), dans lequel ledit partenaire de liaison par détection est marqué ; ledit partenaire de liaison par détection étant en option marqué par un conjugué de phosphatase alcaline.

6. Procédé de la revendication 3(a),
(a) dans lequel ledit deuxième partenaire de liaison est un anticorps monoclonal choisi dans le groupe consistant en 3F4, POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4 et BDI115 ; ou
(b) dans lequel ledit PrP^{c} contient un domaine globulaire à l'intérieur de séquences d'acides aminés correspondant audit noyau résistant aux protéases, ledit deuxième épitope étant situé à l'intérieur dudit domaine globulaire.

7. Procédé de la revendication 6(b), dans lequel ledit deuxième partenaire de liaison est un anticorps monoclonal choisi dans le groupe consistant en POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4 et BDI115.

8. Procédé de la revendication 1 ou 2(a),
(a) dans lequel ladite protéase spécifique de site est immobilisée ; ou
(b) dans lequel ledit inhibiteur de protéase est le fluorure de phénylméthylsulfonyle ; ou
(c) dans lequel ladite étape de dénaturation comprend le traitement dudit échantillon avec un composé de guanidinium ; et comprenant en option en outre une étape de dilution dudit échantillon après ladite étape de dénaturation ; ou
(d) dans lequel ladite étape de dénaturation est mise en oeuvre par exposition dudit échantillon à un pH élevé ou à un faible pH ; et comprenant en option en outre l'étape de neutralisation dudit pH élevé ou dudit faible pH après ladite étape de dénaturation ; ou
(e) dans lequel ledit échantillon est obtenu d'un organisme vivant ou ayant été vivant ; ledit organisme vivant ou ayant été vivant étant en option choisi dans le groupe consistant en les humains, les singes, les hamsters, les bovins, les moutons, les souris, les élans et les cervidés ; ou
(f) dans lequel ledit échantillon provient du groupe consistant en les apports nutritifs, le sang total, les produits sanguins, les fractions sanguines, les composants du sang, le plasma, les plaquettes, le sérum, le liquide céphalorachidien, les organes, les cellules, les tissus cérébraux, les tissus du système nerveux, les tissus musculaires, les tissus gras, la moelle osseuse, l'urine, les larmes, les tissus du système non nerveux, les biopsies, les nécropsies et les instruments contaminés ; ou
(g) dans lequel ledit échantillon dérive du groupe consistant en le sang total, les produits sanguins, les fractions sanguines, les composants du sang, le plasma, les plaquettes, les globules rouges et le sérum ; ou
(h) dans lequel ledit échantillon est obtenu à partir d'une biopsie, d'une autopsie ou d'une nécropsie.

9. Trousse pour détecter la présence d'une forme pathogène de la protéine prion (PrP^{Sc}), qui a un noyau résistant aux protéases et une région à huit répétitions, dans un échantillon que l'on suspecte de contenir ledit PrP^{Sc}, ledit échantillon pouvant, ou non, contenir une forme normale de la protéine prion (PrP^{c}), la trousse comprenant :
(a) au moins une protéase spécifique de site, ladite protéase spécifique de site étant la trypsine ou le S-V8,
i. ledit PrP^{Sc} n'ayant pas de site de clivage pour ladite protéase à l'intérieur de ladite région à huit répétitions ou entre ladite région à huit répétitions et ledit noyau résistant aux protéases, de sorte qu'un fragment de la région amino-proximale, comprenant ladite région à huit répétitions, reste connecté audit noyau résistant aux protéases après une digestion protéolytique du PrP^{Sc} par ladite protéase, qui conduit au clivage dudit PrP^{Sc} sur au moins 90 % de l'ensemble des sites disponibles de clivage par des protéases, et
ii.ledit PrP^{c} ayant au moins un site de clivage disponible pour ladite protéase à l'intérieur de la région d'acides aminés correspondant audit noyau résistant aux protéases ;
(b) en option, un inhibiteur de protéase, qui est capable d'inhiber l'activité de ladite protéase ;
(c) en option, un dénaturant, qui est capable de dénaturer ledit PrP^{Sc},
(d) au moins deux partenaires de liaison, un premier partenaire de liaison et un deuxième partenaire de liaison,
i. ledit premier partenaire de liaison se liant d'une manière spécifique à un premier épitope qui est situé à l'intérieur dudit fragment de la région amino-proximale qui reste connecté audit noyau résistant aux protéases après une digestion protéolytique dudit PrP^{Sc}, ce qui conduit au clivage dudit PrP^{Sc} sur au moins 90 % de l'ensemble des sites disponibles de clivage par des protéases par ladite protéase, et
ii.ledit deuxième partenaire de liaison se liant d'une manière spécifique à un deuxième épitope qui est situé à l'intérieur dudit noyau résistant aux protéases, ledit deuxième épitope se trouvant dans une région dudit PrP^{c} qui est séparée dudit premier épitope après une digestion protéolytique dudit PrP^{c}, qui conduit au clivage dudit PrP^{c} sur au moins 90 % de l'ensemble des sites disponibles de clivage par des protéases par ladite protéase ; et
(e) une notice, pour l'utilisation de ladite trousse dans le but de détecter la présence d'une forme pathogène quelconque de la protéine prion.

10. Trousse de la revendication 9, dans laquelle ledit PrP^{Sc} comprend une séquence choisie parmi SEQ ID N° 1 à 10.

11. Trousse de la revendication 9 ou 10,
(a) dans laquelle ledit premier épitope se trouve à l'intérieur de ladite région à huit répétitions ; ledit premier partenaire de liaison étant en option un anticorps monoclonal choisi dans le groupe consistant en POM2, POM11, POM12, POM14, 3B5, 4F2, 13F10, SAF-15, SAF-31, SAF-32, SAF-33, SAF-34, SAF-35 et SAF-37 ; ou
(b) dans laquelle ledit premier épitope se trouve à l'extérieur de ladite région à huit répétitions ; ledit premier partenaire de liaison étant en option un anticorps monoclonal choisi dans le groupe consistant en BAR210, BAR231 et 14D3 ; ou
(c) dans laquelle ladite protéase spécifique de site est la trypsine ; ou
(d) dans laquelle ladite protéase spécifique de site est le S-V8 ; ledit deuxième partenaire de liaison étant en option un anticorps monoclonal choisi dans le groupe consistant en SAF-53, SAF-54, SAF-60, SAF-61, SAF-66, SAF-69, SAF-70, SAF-75, SAF-76, Pri917, BAR234, Sha31, 11B9, 12F10, 6H4 et POM5 ; ou
(e) dans laquelle lesdits partenaires de liaison sont des aptamères ; ou
(f) dans laquelle lesdits partenaires de liaison sont des anticorps ; lesdits anticorps étant en option des anticorps monoclonaux ; ou
(g) dans laquelle ladite trousse comprend une trousse pour ELISA.

12. Trousse de la revendication 11(g),
(a) dans laquelle ledit premier partenaire de liaison est un partenaire de liaison par capture pour capturer des protéines prions, et ledit deuxième partenaire de liaison étant un partenaire de liaison par détection pour détecter ledit PrP^{Sc} ; ou
(b) dans laquelle ledit deuxième partenaire de liaison est un partenaire de liaison par capture pour capturer des protéines prions, et ledit premier partenaire de liaison étant un partenaire de liaison par détection pour détecter ledit PrP^{Sc}.

13. Trousse de la revendication 12(a) ou 12(b), dans laquelle ledit partenaire de liaison par détection est marqué ; ledit partenaire de liaison par détection étant en option marqué avec un conjugué de phosphatase alcaline.

14. Trousse de la revendication 11(c),
(a) dans laquelle ledit deuxième partenaire de liaison est un anticorps monoclonal choisi dans le groupe consistant en 3F4, POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4 et BDI115 ; ou
(b) dans laquelle ledit PrP^{c} contient un domaine globulaire à l'intérieur de séquences d'acides aminés correspondant audit noyau résistant aux protéases, ledit deuxième épitope étant situé à l'intérieur dudit domaine globulaire ; ledit deuxième partenaire de liaison étant en option un anticorps monoclonal choisi dans le groupe consistant en POM1, POM4, POM5, POM6, POM7, POM8, POM9, POM10, POM13, POM15, POM16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4 et BDI115.

15. Trousse de la revendication 9 ou 10,
(a) dans laquelle ladite protéase spécifique de site est immobilisée ; ou
(b) dans laquelle ledit inhibiteur de protéase en option est le fluorure de phénylméthylsulfonyle ; ou
(c) dans laquelle ledit dénaturant en option est un composé du guanidinium ; ou
(d) dans laquelle ledit dénaturant en option est un réactif basique ou un réactif acide.


### SEQUENCE LISTING

<110> Novartis AG
   Peretz, David
   Yam, Alice
<120> PRION ASSAY
<130> PP028406.0003
<150> US 60/921,951
   <151> 2007-04-04
<150> US 61/066,704
   <151> 2008-02-21
<160> 12
<170> PatentIn version 3.5
<210> 1
   <211> 253
   <212> PRT
   <213> Artificial sequence
<220>
   <223> Human prion protein
<400> 1
<210> 2
   <211> 244
   <212> PRT
   <213> Artificial sequence
<220>
   <223> Monkey prion protein
<400> 2
   Met Ala Asn Leu Gly Cys Trp Met Leu Val Leu Phe Val Ala Thr Trp
   1               5                   10                  15
   Ser Asp Leu Gly Leu Cys Lys Lys Arg Pro Lys Pro Gly Gly Trp Asn
               20                  25                  30
   Thr Gly Gly Ser Arg Tyr Pro Gly Gly Gly Ser Pro Gly Gly Asn Arg
           35                  40                  45
   Tyr Pro Pro Gln Gly Gly Gly Gly Trp Gly Gln Pro His Gly Gly Gly
       50                  55                  60
   Trp Gly Gln Pro His Gly Gly Gly Trp Gly Gln Pro His Gly Gly Gly
   65                  70                  75                  80
   Trp Gly Gln Gly Gly Gly Thr His Asn Gln Trp His Lys Pro Ser Lys
                   85                  90                  95
   Pro Lys Thr Ser Met Lys His Met Ala Gly Ala Ala Ala Ala Gly Ala
               100                 105                 110
   Val Val Gly Gly Leu Gly Gly Tyr Met Leu Gly Ser Ala Met Ser Arg
           115                 120                 125
   Pro Leu Ile His Phe Gly Asn Asp Tyr Glu Asp Arg Tyr Tyr Arg Glu
       130                 135                 140
   Asn Met Tyr Arg Tyr Pro Asn Gln Val Tyr Tyr Arg Pro Val Asp Gln
   145                 150                 155                 160
   Tyr Ser Asn Gln Asn Asn Phe Val His Asp Cys Val Asn Ile Thr Ile
                   165                 170                 175
   Lys Gln His Thr Val Thr Thr Thr Thr Lys Gly Glu Asn Phe Thr Glu
               180                 185                 190
   Thr Asp Val Lys Met Met Glu Arg Val Val Arg Gln Met Cys Ile Thr
           195                 200                 205
   Gln Tyr Glu Lys Glu Ser Gln Ala Tyr Tyr Gln Arg Gly Ser Ser Met
       210                 215                 220
   Val Leu Phe Ser Ser Pro Pro Val Ile Leu Leu Ile Ser Phe Leu Phe
   225                 230                 235                 240
   Leu Ile Val Gly
<210> 3
   <211> 254
   <212> PRT
   <213> Artificial sequence
<220>
   <223> Syrian hamster prion protein
<400> 3
   Met Ala Asn Leu Ser Tyr Trp Leu Leu Ala Leu Phe Val Ala Met Trp
   1 5 10 15
   Thr Asp Val Gly Leu Cys Lys Lys Arg Pro Lys Pro Gly Gly Trp Asn
   20 25 30
   Thr Gly Gly Ser Arg Tyr Pro Gly Gln Gly Ser Pro Gly Gly Asn Arg
   35 40 45
   Tyr Pro Pro Gln Gly Gly Gly Thr Trp Gly Gln Pro His Gly Gly Gly
   50 55 60
   Trp Gly Gln Pro His Gly Gly Gly Trp Gly Gln Pro His Gly Gly Gly
   65 70 75 80
   Trp Gly Gln Pro His Gly Gly Gly Trp Gly Gln Gly Gly Gly Thr His
   85 90 95
   Asn Gln Trp Asn Lys Pro Ser Lys Pro Lys Thr Asn Met Lys His Met
   100 105 110
   Ala Gly Ala Ala Ala Ala Gly Ala Val Val Gly Gly Leu Gly Gly Tyr
   115 120 125
   Met Leu Gly Ser Ala Met Ser Arg Pro Met Met His Phe Gly Asn Asp
   130 135 140
   Trp Glu Asp Arg Tyr Tyr Arg Glu Asn Met Asn Arg Tyr Pro Asn Gln
   145 150 155 160
   Val Tyr Tyr Arg Pro Val Asp Gln Tyr Asn Asn Gln Asn Asn Phe Val
   165 170 175
   His Asp Cys Val Asn Ile Thr Ile Lys Gln His Thr Val Thr Thr Thr
   180 185 190
   Thr Lys Gly Glu Asn Phe Thr Glu Thr Asp Ile Lys Ile Met Glu Arg
   195 200 205
   Val Val Glu Gln Met Cys Thr Thr Gln Tyr Gln Lys Glu Ser Gln Ala
   210 215 220
   Tyr Tyr Asp Gly Arg Arg Ser Ser Ala Val Leu Phe Ser Ser Pro Pro
   225 230 235 240
   Val Ile Leu Leu Ile Ser Phe Leu Ile Phe Leu Met Val Gly
   245 250
<210> 4
   <211> 264
   <212> PRT
   <213> Artificial Sequence
<220>
   <223> Bovine prion protein
<220>
   <221> MISC_FEATURE
   <222> (46)..(46)
   <223> xaa can be any naturally occurring amino acid
<220>
   <221> MISC_FEATURE
   <222> (154)..(154)
   <223> Xaa can be any naturally occurring amino acid
<400> 4
   Met Val Lys Ser His Ile Gly Ser Trp Ile Leu Val Leu Phe Val Ala
   1               5                   10                  15
   Met Trp Ser Asp Val Gly Leu Cys Lys Lys Arg Pro Lys Pro Gly Gly
               20                  25                  30
   Gly Trp Asn Thr Gly Gly Ser Arg Tyr Pro Gly Gln Gly Xaa Pro Gly
           35                  40                  45
   Gly Asn Arg Tyr Pro Pro Gln Gly Gly Gly Gly Trp Gly Gln Pro His
       50                  55                  60
   Gly Gly Gly Trp Gly Gln Pro His Gly Gly Gly Trp Gly Gln Pro His
   65                  70                  75                  80
   Gly Gly Gly Trp Gly Gln Pro His Gly Gly Gly Trp Gly Gln Pro His
                   85                  90                  95
   Gly Gly Gly Gly Trp Gly Gln Gly Gly Thr His Gly Gln Trp Asn Lys
               100                 105                 110
   Pro Ser Lys Pro Lys Thr Asn Met Lys His Val Ala Gly Ala Ala Ala
           115                 120                 125
   Ala Gly Ala Val Val Gly Gly Leu Gly Gly Tyr Met Leu Gly Ser Ala
       130                 135                 140
   Met Ser Arg Pro Leu Ile His Phe Gly Xaa Asp Tyr Glu Asp Arg Tyr
   145                 150                 155                 160
   Tyr Arg Glu Asn Met His Arg Tyr Pro Asn Gln Val Tyr Tyr Arg Pro
                   165                 170                 175
   Val Asp Gln Tyr Ser Asn Gln Asn Asn Phe Val His Asp Cys Val Asn
               180                 185                 190
   Ile Thr Val Lys Glu His Thr Val Thr Thr Thr Thr Lys Gly Glu Asn
           195                 200                 205
   Phe Thr Glu Thr Asp Ile Lys Met Met Glu Arg Val Val Glu Gln Met
   210 215 220
   Cys Ile Thr Gln Tyr Gln Arg Glu Ser Gln Ala Tyr Tyr Gln Arg Gly
   225 230 235 240
   Ala Ser Val Ile Leu Phe Ser Ser Pro Pro Val Ile Leu Leu Ile Ser
   245 250 255
   Phe Leu Ile Phe Leu Ile Val Gly
   260
<210> 5
   <211> 256
   <212> PRT
   <213> Artificial sequence
<220>
   <223> sheep prion protein
<400> 5
   Met Val Lys Ser His Ile Gly Ser Trp Ile Leu Val Leu Phe Val Ala
   1 5 10 15
   Met Trp Ser Asp Val Gly Leu Cys Lys Lys Arg Pro Lys Pro Gly Gly
   20 25 30
   Gly Trp Asn Thr Gly Gly Ser Arg Tyr Pro Gly Gln Gly Ser Pro Gly
   35 40 45
   Gly Asn Arg Tyr Pro Pro Gln Gly Gly Gly Gly Trp Gly Gln Pro His
   50 55 60
   Gly Gly Gly Trp Gly Gln Pro His Gly Gly Gly Trp Gly Gln Pro His
   65 70 75 80
   Gly Gly Gly Trp Gly Gln Pro His Gly Gly Gly Gly Trp Gly Gln Gly
   85 90 95
   Gly Ser His Ser Gln Trp Asn Lys Pro Ser Lys Pro Lys Thr Asn Met
   100 105 110
   Lys His Val Ala Gly Ala Ala Ala Ala Gly Ala Val Val Gly Gly Leu
   115 120 125
   Gly Gly Tyr Met Leu Gly Ser Ala Met Ser Arg Pro Leu Ile His Phe
   130 135 140
   Gly Asn Asp Tyr Glu Asp Arg Tyr Tyr Arg Glu Asn Met Tyr Arg Tyr
   145 150 155 160
   Pro Asn Gln Val Tyr Tyr Arg Pro Val Asp Arg Tyr Ser Asn Gln Asn
   165 170 175
   Asn Phe Val His Asp Cys Val Asn Ile Thr Val Lys Gln His Thr Val
   180 185 190
   Thr Thr Thr Thr Lys Gly Glu Asn Phe Thr Glu Thr Asp Ile Lys Ile
   195 200 205
   Met Glu Arg Val Val Glu Gln Met Cys Ile Thr Gln Tyr Gln Arg Glu
   210 215 220
   Ser Gln Ala Tyr Tyr Gln Arg Gly Ala Ser Val Ile Leu Phe Ser Ser
   225 230 235 240
   Pro Pro Val Ile Leu Leu Ile Ser Phe Leu Ile Phe Leu Ile Val Gly
   245 250 255
<210> 6
   <211> 254
   <212> PRT
   <213> Artificial Sequence
<220>
   <223> Mouse prion protein
<400> 6
   Met Ala Asn Leu Gly Tyr Trp Leu Leu Ala Leu Phe Val Thr Met Trp
   1 5 10 15
   Thr Asp Val Gly Leu Cys Lys Lys Arg Pro Lys Pro Gly Gly Trp Asn
   20 25 30
   Thr Gly Gly Ser Arg Tyr Pro Gly Gln Gly Ser Pro Gly Gly Asn Arg
   35 40 45
   Tyr Pro Pro Gln Gly Gly Thr Trp Gly Gln Pro His Gly Gly Gly Trp
   50 55 60
   Gly Gln Pro His Gly Gly Ser Trp Gly Gln Pro His Gly Gly Ser Trp
   65 70 75 80
   Gly Gln Pro His Gly Gly Gly Trp Gly Gln Gly Gly Gly Thr His Asn
   85 90 95
   Gln Trp Asn Lys Pro Ser Lys Pro Lys Thr Asn Leu Lys His Val Ala
   100 105 110
   Gly Ala Ala Ala Ala Gly Ala Val Val Gly Gly Leu Gly Gly Tyr Met
   115 120 125
   Leu Gly Ser Ala Met Ser Arg Pro Met Ile His Phe Gly Asn Asp Trp
   130 135 140
   Glu Asp Arg Tyr Tyr Arg Glu Asn Met Tyr Arg Tyr Pro Asn Gln Val
   145 150 155 160
   Tyr Tyr Arg Pro Val Asp Gln Tyr Ser Asn Gln Asn Asn Phe Val His
   165 170 175
   Asp Cys Val Asn Ile Thr Ile Lys Gln His Thr Val Thr Thr Thr Thr
   180 185 190
   Lys Gly Glu Asn Phe Thr Glu Thr Asp Val Lys Met Met Glu Arg Val
   195 200 205
   Val Glu Gln Met Cys Val Thr Gln Tyr Gln Lys Glu Ser Gln Ala Tyr
   210 215 220
   Tyr Asp Gly Arg Arg Ser Ser Ser Thr Val Leu Phe Ser Ser Pro Pro
   225 230 235 240
   Val Ile Leu Leu Ile Ser Phe Leu Ile Phe Leu Ile Val Gly
   245 250
<210> 7
   <211> 256
   <212> PRT
   <213> Artificial Sequence
<220>
   <223> Elk prion protein
<400> 7
   Met Val Lys Ser His Ile Gly Ser Trp Ile Leu Val Leu Phe Val Ala
   1 5 10 15
   Met Trp Ser Asp Val Gly Leu Cys Lys Lys Arg Pro Lys Pro Gly Gly
   20 25 30
   Gly Trp Asn Thr Gly Gly Ser Arg Tyr Pro Gly Gln Gly Ser Pro Gly
   35 40 45
   Gly Asn Arg Tyr Pro Pro Gln Gly Gly Gly Gly Trp Gly Gln Pro His
   50 55 60
   Gly Gly Gly Trp Gly Gln Pro His Gly Gly Gly Trp Gly Gln Pro His
   65 70 75 80
   Gly Gly Gly Trp Gly Gln Pro His Gly Gly Gly Gly Trp Gly Gln Gly
   85 90 95
   Gly Thr His Ser Gln Trp Asn Lys Pro Ser Lys Pro Lys Thr Asn Met
   100 105 110
   Lys His Val Ala Gly Ala Ala Ala Ala Gly Ala Val Val Gly Gly Leu
   115 120 125
   Gly Gly Tyr Met Leu Gly Ser Ala Met Ser Arg Pro Leu Ile His Phe
   130 135 140
   Gly Asn Asp Tyr Glu Asp Arg Tyr Tyr Arg Glu Asn Met Tyr Arg Tyr
   145 150 155 160
   Pro Asn Gln Val Tyr Tyr Arg Pro Val Asp Gln Tyr Asn Asn Gln Asn
   165 170 175
   Thr Phe Val His Asp Cys Val Asn Ile Thr Val Lys Gln His Thr Val
   180 185 190
   Thr Thr Thr Thr Lys Gly Glu Asn Phe Thr Glu Thr Asp Ile Lys Met
   195 200 205
   Met Glu Arg Val Val Glu Gln Met Cys Ile Thr Gln Tyr Gln Arg Glu
   210 215 220
   Ser Glu Ala Tyr Tyr Gln Arg Gly Ala Ser Val Ile Leu Phe Ser Ser
   225 230 235 240
   Pro Pro Val Ile Leu Leu Ile Ser Phe Leu Ile Phe Leu Ile Val Gly
   245 250 255
<210> 8
   <211> 256
   <212> PRT
   <213> Artificial Sequence
<220>
   <223> Fallow deer prion protein
<400> 8
   Met Val Lys Ser His Ile Gly Ser Trp Ile Leu Val Leu Phe Val Ala
   1 5 10 15
   Met Trp Ser Asp Val Gly Leu Cys Lys Lys Arg Pro Lys Pro Gly Gly
   20 25 30
   Gly Trp Asn Thr Gly Gly Ser Arg Tyr Pro Gly Gln Gly Ser Pro Gly
   35 40 45
   Gly Asn Arg Tyr Pro Pro Gln Gly Gly Gly Gly Trp Gly Gln Pro His
   50 55 60
   Gly Gly Gly Trp Gly Gln Pro His Gly Gly Gly Trp Gly Gln Pro His
   65 70 75 80
   Gly Gly Gly Trp Gly Gln Pro His Gly Gly Gly Gly Trp Gly Gln Gly
   85 90 95
   Gly Thr His Ser Gln Trp Asn Lys Pro Ser Lys Pro Lys Thr Asn Met
   100 105 110
   Lys His Val Ala Gly Ala Ala Ala Ala Gly Ala Val Val Gly Gly Leu
   115 120 125
   Gly Gly Tyr Met Leu Gly Ser Ala Met Asn Arg Pro Leu Ile His Phe
   130 135 140
   Gly Asn Asp Tyr Glu Asp Arg Tyr Tyr Arg Glu Asn Met Tyr Arg Tyr
   145 150 155 160
   Pro Asn Gln Val Tyr Tyr Arg Pro Val Asp Gln Tyr Asn Asn Gln Asn
   165 170 175
   Thr Phe Val His Asp Cys Val Asn Ile Thr Val Lys Gln His Thr Val
   180 185 190
   Thr Thr Thr Thr Lys Gly Glu Asn Phe Thr Glu Thr Asp Ile Lys Met
   195 200 205
   Met Glu Arg Val Val Glu Gln Met Cys Ile Thr Gln Tyr Gln Arg Glu
   210 215 220
   Ser Glu Ala Tyr Tyr Gln Arg Gly Ala Ser Val Ile Leu Phe Ser Ser
   225 230 235 240
   Pro Pro Val Ile Leu Leu Ile Ser Phe Leu Ile Phe Leu Ile Val Gly
   245 250 255
<210> 9
   <211> 256
   <212> PRT
   <213> Artificial Sequence
<220>
   <223> Mule deer prion protein
<400> 9
   Met Val Lys Ser His Ile Gly Ser Trp Ile Leu Val Leu Phe Val Ala
   1               5                   10                  15
   Met Trp Ser Asp Val Gly Leu Cys Lys Lys Arg Pro Lys Pro Gly Gly
               20                  25                  30
   Gly Trp Asn Thr Gly Gly Ser Arg Tyr Pro Gly Gln Gly Ser Pro Gly
           35                  40                  45
   Gly Asn Arg Tyr Pro Pro Gln Gly Gly Gly Gly Trp Gly Gln Pro His
       50                  55                  60
   Gly Gly Gly Trp Gly Gln Pro His Gly Gly Gly Trp Gly Gln Pro His
   65                  70                  75                  80
   Gly Gly Gly Trp Gly Gln Pro His Gly Gly Gly Gly Trp Gly Gln Gly
                   85                  90                  95
   Gly Thr His Ser Gln Trp Asn Lys Pro Ser Lys Pro Lys Thr Asn Met
               100                 105                 110
   Lys His Val Ala Gly Ala Ala Ala Ala Gly Ala Val Val Gly Gly Leu
           115                 120                 125
   Gly Gly Tyr Met Leu Gly Ser Ala Met Ser Arg Pro Leu Ile His Phe
       130                 135                 140
   Gly Asn Asp Tyr Glu Asp Arg Tyr Tyr Arg Glu Asn Met Tyr Arg Tyr
   145                 150                 155                 160
   Pro Asn Gln Val Tyr Tyr Arg Pro Val Asp Gln Tyr Asn Asn Gln Asn
                   165                 170                 175
   Thr Phe Val His Asp Cys Val Asn Ile Thr Val Lys Gln His Thr Val
               180                 185                 190
   Thr Thr Thr Thr Lys Gly Glu Asn Phe Thr Glu Thr Asp Ile Lys Met
           195                 200                 205
   Met Glu Arg Val Val Glu Gln Met Cys Ile Thr Gln Tyr Gln Arg Glu
       210                 215                 220
   Ser Gln Ala Tyr Tyr Gln Arg Gly Ala Ser Val Ile Leu Phe Ser Ser
   225                 230                 235                 240
   Pro Pro Val Ile Leu Leu Ile Ser Phe Leu Ile Phe Leu Ile Val Gly
                   245                 250                 255
<210> 10
   <211> 256
   <212> PRT
   <213> Artificial Sequence
<220>
   <223> white-tailed deer prion protein
<400> 10
   Met Val Lys Ser His Ile Gly Ser Trp Ile Leu Val Leu Phe Val Ala
   1               5                   10                  15
   Met Trp Ser Asp Val Gly Leu Cys Lys Lys Arg Pro Lys Pro Gly Gly
               20                  25                  30
   Gly Trp Asn Thr Gly Gly Ser Arg Tyr Pro Gly Gln Gly Ser Pro Gly
           35                  40                  45
   Gly Asn Arg Tyr Pro Pro Gln Gly Gly Gly Gly Trp Gly Gln Pro His
       50                  55                  60
   Gly Gly Gly Trp Gly Gln Pro His Gly Gly Gly Trp Gly Gln Pro His
   65                  70                  75                  80
   Gly Gly Gly Trp Gly Gln Pro His Gly Gly Gly Gly Trp Gly Gln Gly
                   85                  90                  95
   Gly Thr His Ser Gln Trp Asn Lys Pro Ser Lys Pro Lys Thr Asn Met
               100                 105                 110
   Lys His Val Ala Gly Ala Ala Ala Ala Gly Ala Val Val Gly Gly Leu
           115                 120                 125
   Gly Gly Tyr Met Leu Gly Ser Ala Met Ser Arg Pro Leu Ile His Phe
       130                 135                 140
   Gly Asn Asp Tyr Glu Asp Arg Tyr Tyr Arg Glu Asn Met Tyr Arg Tyr
   145                 150                 155                 160
   Pro Asn Gln Val Tyr Tyr Arg Pro Val Asp Gln Tyr Asn Asn Gln Asn
                   165                 170                 175
   Thr Phe Val His Asp Cys Val Asn Ile Thr Val Lys Gln His Thr Val
               180                 185                 190
   Thr Thr Thr Thr Lys Gly Glu Asn Phe Thr Glu Thr Asp Ile Lys Met
           195                 200                 205
   Met Glu Arg Val Val Glu Gln Met Cys Ile Thr Gln Tyr Gln Arg Glu
       210                 215                 220
   Ser Gln Ala Tyr Tyr Gln Arg Gly Ala Ser Val Ile Leu Phe Ser Ser
   225                 230                 235                 240
   Pro Pro Val Ile Leu Leu Ile Ser Phe Leu Ile Phe Leu Ile Val Gly
                   245                 250                 255
<210> 11
   <211> 9
   <212> PRT
   <213> Artificial Sequence
<220>
   <223> Octarepeat sequence
<220>
   <221> MISC_FEATURE
   <222> (7) .. (7)
   <223> Xaa = Gly or Ser
<220>
   <221> MISC_FEATURE
   <222> (8)..(8)
   <223> Gly may br present or absent
<400> 11
   Gly Gln Pro His Gly Gly Xaa Gly Trp
   1               5
<210> 12
   <211> 9
   <212> PRT
   <213> Artificial Sequence
<220>
   <223> Octarepeat sequence
<220>
   <221> MISC_FEATURE
   <222> (2) .. (2)
   <223> Xaa = His or Gln
<220>
   <221> MISC_FEATURE
   <222> (6) .. (6)
   <223> Thr may br present or absent
<400> 12
   Pro Xaa Gly Gly Gly Thr Trp Gly Gln
   1               5